# EUROPÄISCHE PATENTSCHRIFT

(11) **EP 3 842 052 B1**
(45) Veröffentlichungstag und Bekanntmachung des Hinweises auf die Patenterteilung: **28.05.2025**
(21) Anmeldenummer: 19219356.3
(22) Anmeldetag: 23.12.2019
(51) Int. Cl.: A61K 36/062, A61P 31/04

(54) **MITTEL UND VERFAHREN ZUR STABILISIERUNG DER DARMFLORA UND VERBESSERUNG DER HYGIENE**
COMPOSITION AND METHODS FOR STABILISING INTESTINAL FLORA AND IMPROVING HYGIENE
AGENT ET PROCÉDÉ DE STABILISATION DE LA FLORE INTESTINALE ET D'AMÉLIORATION DE L'HYGIÈNE

(43) Veröffentlichungstag der Anmeldung: 30.06.2021
(73) Patentinhaber: Senzyme GmbH, 53840 Troisdorf (DE)
(72) Erfinder: Janßen, Martina Iris, 53639 Königswinter (DE); Rühenbeck, Birgit Liliane, 53225 Bonn (DE); Lenz, Carl Jürgen, 53347 Alfter (DE)
(74) Vertreter: Hauck Patentanwaltspartnerschaft mbB

(56) Entgegenhaltungen:
- WO-A1-95/33836
- WO-A1-97/06775
- CN-A- 109 679 856
- US-A- 6 080 391
- DATABASE WPI Week 201957, Derwent World Patents Index; AN 2019-41101J, XP002798936

## Beschreibung

Die Erfindung bezieht sich auf Verwendungen eines bakteriziden Mittels zur Leistungssteigerung oder zur Förderung des Wohlbefindens von Wirbeltieren und/oder Menschen, als Desinfektionsmittel und/oder als Konservierungsmittel im Umfeld von Wirbeltieren und/oder Menschen oder als Mittel zur Körperhygiene bei Wirbeltieren und/oder Menschen.

### Hintergrund

Der Verdauungstrakt von Wirbeltieren und des Menschen stellt ein Organsystem dar, in dem nicht nur Futter- beziehungsweise Nahrungsmittel durch mechanische Einwirkungen zerkleinert, transportiert und durch körpereigene, enzymhaltige Flüssigkeiten verdaut werden, sondern dem auch eine besondere gesundheitliche Bedeutung zukommt. Dies gilt vor allem für den Darm als Teil des Verdauungstraktes, wobei hier unter "Darm" die Gesamtheit aller Darmabschnitte wie Zwölffingerdarm (Duodenum), Leerdarm (Jejunum), Hüftdarm (Ileum) - wobei diese drei Darmabschnitte gemeinsam auch als Dünndarm bezeichnet werden, Dickdarm (Colon) und Enddarm (Rektum), nebst Darmanhängen wie den Blinddärmen (Caeca) verstanden wird. Der Aufbau des Darms, die Darmabschnitte und die Darmanhänge können zwischen den einzelnen Klassen der Wirbeltiere (Säugetiere und Mensch, Vögel, Reptilien, Amphibien und Fische) sowie auch innerhalb der Klassen variieren, was jedoch hier nicht im Detail ausgeführt werden soll. Die gesundheitliche Bedeutung des Darms erwächst unter anderem aus der Vielzahl an Mikroorganismen, die von außerhalb in den Darm gelangen und ihn besiedeln (der Darm kann deshalb zu Recht als mikrobielles Ökosystem beschrieben werden) und die nicht nur an den Verdauungsprozessen beteiligt sind, sondern die auch das Immunsystem beeinflussen und Immunreaktionen hervorrufen können. Sie tragen in ihrer Gesamtheit wesentlich zum physiologischen Gleichgewicht des Darms bei. Störungen dieses Gleichgewichts, wie sie durch Imbalancen innerhalb der "Darmflora" (der Gesamtheit aller im Darm befindlichen Mikroorganismen) oder durch mikrobielle Darminfektionen und in der Folge durch entzündliche Darmerkrankungen hervorgerufen werden, haben erhebliche Auswirkungen auf den Gesamtzustand, die Leistungsfähigkeit und das Wohlbefinden des Organismus.

Die Darmflora konstituierende Mikroorganismengruppen sind Bakterien, Archaen, Hefen, Pilze und Protozoen, wobei die Bakterien den überwiegenden Anteil der Darmflora bilden. Die Bakterien lassen sich aufgrund der Struktur ihrer Zellwände in zwei große Gruppen einteilen: "Gram-positive" Bakterien besitzen eine auf ihrer lipiddoppelschichtigen Zellmembran aufgelagerte, mehrschichtige Hülle aus Peptidoglycan, wobei es sich bei "Peptigoglycan" um ein aus Zuckern und Aminosäuren zusammengesetztes Makromolekül handelt. Bei "gram-negativen" Bakterien ist die Peptidoglycanhülle nur dünnschichtig ausgebildet. Darauf aufgelagert ist bei gram-negativen Bakterien eine zweite Lipiddoppelschicht.

Generell kann ein im Vergleich zum natürlichen Zustand verschobenes Übergewicht der einen oder anderen Bakteriengruppe eine Störung der Darmphysiologie verursachen. Innerhalb der gram-positiven und gram-negativen Bakterien finden sich zudem zahlreiche pathogene, das heißt krankheitserregende Stämme. Bekannte gram-negative pathogene Stämme finden sich zum Beispiel innerhalb der Gattung *Salmonella* und der Art *Escherichia coli.* Bei den gram-positiven Bakterien finden sich pathogene Stämme beispielsweise innerhalb der Gattungen *Bacillus, Clostridium, Enterococcus, Staphylococcus* und *Streptococcus.*

Bakterielle Darminfektionen werden traditionell mit Antibiotika behandelt, die gegenüber bestimmten Bakterien bakterizid oder bakteriostatisch wirken können. Unter "Antibiotika" im herkömmlichen Sinne sollen hier deshalb Mittel verstanden werden, die Vertreter von Wirkstoffgruppen gegen Bakterien wie beispielsweise Penicilline, Cephalosporine, Tetracycline, Makrolide und Sulfonamide enthalten und die in Form von Arzneimitteln (Pharmazeutika) verabreicht werden. "Bakterizid" bedeutet, dass die Bakterien durch das verabreichte Antibiotikum abgetötet werden, wohingegen "bakteriostatisch" bedeutet, dass das Wachstum und die Vermehrungsfähigkeit der Bakterien gehemmt werden. Nach der Trennung von dem bakteriostatisch wirkenden Antibiotikum können sich die Bakterien wieder vermehren. Bakterizide und bakteriostatische Wirkungen werden im Folgenden auch als "antibakterielle" Wirkungen zusammengefasst. Antibiotika wirken gezielt und spezifisch auf bestimmte zelluläre und molekulare Strukturen derjenigen Bakterien, gegen die sie gerichtet sind. Durch diese Zielstrukturen wird eine selektive Toxizität gegenüber den Bakterien erreicht, sodass die Zellen des Wirtsorganismus nicht geschädigt werden. Antibiotika können zu therapeutischen Zwecken oder aus Gründen der Prophylaxe gegen Krankheiten eingesetzt werden. In der Nutztierhaltung wurden Antibiotika lange Zeit zur Leistungsförderung auch ohne veterinärmedizinische Indikation angewendet. Diese Praxis ist in der Europäischen Union seit 2006 verboten.

Bakterien können eine intrinsische Resistenz gegen Antibiotika dadurch besitzen, dass die entsprechenden Zielstrukturen nicht vorhanden sind. Bakterien können gegen Antibiotika aber auch resistent werden (erworbene Resistenz). Der erworbenen Resistenz liegt ein Prozess von Mutation und Selektion unter dem Selektionsdruck des Vorhandenseins eines oder mehrerer Antibiotika im jeweiligen Lebensraum der Bakterien zugrunde. Darüber hinaus können dermaßen erworbene Resistenzen von einer auf eine andere Bakterienart genetisch übertragen werden.

Die Erkenntnis, dass der über Jahrzehnte hinweg erfolgte, teils übermäßige Einsatz von Antibiotika sowohl in der Tierhaltung als auch in humanmedizinischen Anwendungen weltweit zur Entstehung und vor allem zur stetigen Zunahme pathogener Bakterien mit Resistenzen gegenüber Antibiotika wesentlich beigetragen hat, hat zu großer Besorgnis und zu internationalen Bestrebungen geführt, den Einsatz herkömmlich verwendeter Antibiotika deutlich zu reduzieren. Antibiotika können zudem gesundheitliche Nebenwirkungen haben, wie zum Beispiel Durchfall hervorrufen oder immunsupressive Effekte verursachen, die dann Sekundärinfektionen mit anderen pathogenen Mikroorganismen (beispielsweise anderen Bakterien, Hefen oder Pilzen) zur Folge haben können. Dies alles hat dazu geführt, die Suche nach Alternativen zum Einsatz von Antibiotika sowohl im Bereich der Tierhaltung als auch im humanen Bereich zu intensivieren.

Zwei wichtige Ansätze zur Erreichung des Ziels einer nachhaltigen Reduktion des Antibiotika-Einsatzes sind 1) die Stabilisierung der Darmflora durch alternative Mittel beziehungsweise Wirkstoffe und 2) die Verbesserung der Hygiene.

Zur Stabilisierung der Darmflora werden nach dem derzeitigen Stand der Technik typischerweise die folgenden Gruppen alternativer Mittel, beziehungsweise Wirkstoffe angewendet: Probiotika, Präbiotika, Phytogene, organische Säuren und Mineralstoffe.

"Probiotika" sind Mittel, die lebende Mikroorganismen (sogenannte probiotische Bakterien wie Milchsäurebakterien oder auch probiotische Hefen) enthalten, welche den Darm besiedeln und auf diese Weise die Darmflora positiv beeinflussen. Dies kann zum Beispiel dadurch geschehen, dass sie pathogene Keime verdrängen (kompetitiver Effekt), wobei das zugrundeliegende Wirkprinzip eine Konkurrenz um Lebensraum und Nährstoffe ist. Probiotische Bakterien können zudem antibakteriell wirkende Peptide bilden und Stoffwechselprodukte, wie kurzkettige Fettsäuren, ausscheiden, die positive Effekte auf das Immunsystem entfalten können, beispielsweise indem sie die Bildung von immunologisch aktiven Signalstoffen wie Cytokinen, Interleukinen und anderen modulieren. Zu den probiotischen Mitteln gehören auch Sporen, also Dauerstadien von Bakterienzellen, die nach der Applikation im Darm auskeimen und zu aktiven Zellen werden, welche sich anschließend vermehren.

"Präbiotika" sind Mittel, die Wirkstoffe enthalten, welche das Wachstum probiotischer Bakterien im Darm fördern. Präbiotische Wirkstoffe können beispielsweise bestimmte Zucker oder Oligosaccharide (das sind aus wenigen, typischerweise 2-7, Zuckerbausteinen bestehende Molekülketten) sein, die vom Organismus, dem die präbiotischen Wirkstoffe verabreicht werden, selbst nicht verwertet werden können, die aber durch die Bakterien im Darm, üblicherweise in den hinteren Darmabschnitten oder in den Darmanhängen höherer Tiere und des Menschen, verstoffwechselbar sind.

"Phytogene" sind Mittel, die Wirkstoffe enthalten, welche aus Pflanzen oder Pflanzenteilen gewonnen werden und die im Darm einen antibakteriellen Effekt entfalten können. Phytogene Wirkstoffe sind zum Beispiel bestimmte pflanzliche Öle (wie Thymol, Carvacrol, Nelkenöl und Zimtöl) oder gesättigte, mittelkettige Fettsäuren mit 6-12 Kohlenstoffatomen. Letztere sind zum Beispiel in Kokosöl enthalten.

"Organische Säuren" umfassen kurzkettige, gesättigte Fettsäuren wie zum Beispiel Propionsäure und Buttersäure oder andere niedermolekulare Säuren wie beispielsweise Benzoesäure und Zitronensäure. Sie sind vor allem auch als Konservierungsstoffe, beispielsweise für Futter- und Nahrungsmittel, und folglich als Mittel zur Sicherstellung des hygienischen Status von Materialien bekannt. Organische Säuren wirken auf unterschiedliche Weise antibakteriell. Propionsäure beispielsweise stört intrazellulär den Kohlehydratstoffwechsel und die DNA-Synthese. Benzoesäure hemmt intrazellulär Enzyme, die reaktive Sauerstoffspezies (beispielsweise bestimmte Radikale) abbauen und damit am zelleigenen Entgiftungsprozess beteiligt sind.

"Mineralstoffe", die als Nährstoffe (Makroelemente und Spurenelemente) dienen, können ebenfalls direkt auf die Darmflora einwirken. Ein Beispiel hierfür ist Zink, welches in Form von Zinkoxid in geeigneten Formulierungen und bereits subpharmakologischen Dosierungen eine bakterizide Wirkung auf Risikokeime, wie beispielsweise *Escherichia coli,* besitzt.

Es gibt nun verschiedene Nachteile der oben aufgeführten Mittel zur Stabilisierung der Darmflora nach dem Stand der Technik.

So wirken Probiotika und Präbiotika grundsätzlich nur indirekt auf die Darmflora. Zudem ist eine Differenzierung der Wirkung auf gram-positive oder gram-negative Bakterien nicht möglich, zumal typische probiotische Bakterien (wie zum Beispiel Arten der Gattungen *Bacillus, Lactobacillus, Enterococcus* und *Bifidobacterium*) selbst gram-positiv sind. Auch Präbiotika wirken lediglich indirekt, indem sie das Wachstum probiotischer Bakterien fördern. Die von den typischerweise als Probiotika eingesetzten Milchsäurebakterien produzierte Milchsäure kann in größeren Mengen, das heißt, wenn sich die probiotischen Bakterien übermäßig vermehren, im Dünndarm resorbiert werden und gesundheitliche Schäden nach sich ziehen. Grundsätzlich ist die Vermehrung probiotischer Bakterien im Darm nicht kontrollierbar. Im Falle einer Übervermehrung müssen sogar Antibiotika verabreicht werden, was dann im besonderen Maße kontra-indikativ ist. Inzwischen ist auch bekannt, dass eine zu hohe Besiedlung des Darms, insbesondere des Dünndarms, mit Bakterien negative gesundheitliche Eigenschaften haben kann.

Phytogene wirken häufig gegen gram-negative Bakterien, wie Arten der Gattung *Salmonella* oder *Escherichia coli.* In höheren Konzentrationen können Phytogene, wenn sie Futter- oder Nahrungsmitteln zugesetzt werden, Veränderungen im Geschmack oder im Geruch hervorrufen und so die Futter- beziehungsweise Nahrungsaufnahme beeinträchtigen. Viele Phytogene sind dezidierte Aromastoffe. Mittelkettige Fettsäuren wirken zwar gegen gram-positive Bakterien, ihre Wirkung hängt jedoch stark von der jeweiligen Zusammensetzung der Säuremischung ab.

Auch organische Säuren können den Geschmack und Geruch von Futter- bzw. Lebensmitteln negativ beeinflussen. Zudem können sie toxisch wirken, was den Umgang mit diesen Stoffen sehr erschwert und besondere Sicherheitsvorkehrungen in der technischen Verarbeitung und der Anwendung notwendig macht.

Zink ist ein Schwermetall. Es schädigt, wenn es beispielsweise aufgrund unzureichender Resorption über Fäkalien in die Umgebung gelangt, die Umwelt. Deswegen sehen aktuelle Bestrebungen vor, den Einsatz von Zink in der Nutztierhaltung zu beschränken.

Aufgrund der beschriebenen Nachteile besteht grundsätzlich ein großer Bedarf an weiteren Mitteln für die Stabilisierung der Darmflora, insbesondere durch Modulation des Anteils gram-positiver Bakterien im Darm.

Als zweiter wichtiger Ansatz zur nachhaltigen Reduzierung des Einsatzes von Antibiotika wurde die Verbesserung der Hygiene genannt. Unter "Hygiene" sollen hier allgemein Maßnahmen verstanden werden, welche die Belastung von Materialien (feste Stoffe und ihre Oberflächen sowie Flüssigkeiten) im Umfeld von Tier und Mensch sowie der äußeren und inneren Körperoberflächen mit risikobehafteten oder krankheitsverursachenden Mikroorganismen mindern, in dem sie "antimikrobiell" wirken, das heißt die Mikroorganismen abtöten ("biozide" Wirkung) oder im Wachstum und der Vermehrungsfähigkeit hemmen ("biostatische") Wirkung. Hierzu gehören vor allem Maßnahmen zur "Desinfektion" und "Konservierung" sowie zur Körperreinigung. Entsprechend dieser Funktionen spricht man von "Desinfektionsmitteln" und "Konservierungsmitteln" sowie von "Hygienemitteln".

Desinfektionsmittel wirken gegen verschiedene Arten von Mikroorganismen biozid und liegen aus praktischen Gründen in der Regel in flüssiger oder gasförmiger Form vor. Sie sind häufig Bestandteil von "Reinigungsmitteln" und enthalten meist anorganische oder organische Substanzen, seltener biochemische Substanzen, als Wirkstoffe. Beispiele für anorganische und organische Wirkstoffe sind Alkohole (wie Ethanol und 1-Propanol), Aldehyde (wie Formaldehyd), Oxidationsmittel (wie Peressigsäure, Chlor, Chlordioxid, Wasserstoffperoxid, Natriumhypochlorid, Ozon oder Iod), Phenole (wie Chlorxylenol) und Tenside (wie Cetyltrimethylammoniumbromid).

Beispielhaft für eine anorganische Substanz zur Desinfektion sei hier Silber genannt. Silber kann zudem zur desinfizierenden Beschichtung von Oberflächen von Materialien verwendet werden. Oberflächen, die durch eine geeignete Beschichtung eine biozide Wirkung besitzen, werden unter anderem für chirurgische Bestecke verwendet. Bei Kontakt mit der Oberfläche werden die Mikroorganismen abgetötet. Neben Silber sind zum Beispiel auch Kupfer, quartäre Ammoniumverbindungen und Organosilane zur bioziden Oberflächenbeschichtung geeignet. Organosilane wirken dadurch bakterizid, dass sie die äußere Membran gram-negativer Bakterien mechanisch beschädigen. Auf diese Weise verhindern sie die Adhäsion der Bakterien auf der Oberfläche und töten die Bakterien zugleich ab. Auch andere, besondere Arten der Nanostrukturierung von Oberflächen können den Oberflächen eine biozide Wirkung verleihen.

Konservierungsmittel erhöhen die Haltbarkeit von Futtermitteln, Nahrungsmitteln, Kosmetika, Arzneimitteln, Holz, Farben und Beschichtungen, in dem sie den Bewuchs mit Mikroorganismen unterdrücken. Als Konservierungsmittel mit biozider oder biostatischer Wirkung werden häufig organische Säuren wie Benzoesäure und Citronensäure und ihre Salze, Schwefelverbindungen wie Schwefeldioxid und Sulfite, Stickstoffverbindungen wie Nitrate und Nitrite, Parabene usw. verwendet.

Antimikrobielle Wirkstoffe sind auch Bestandteil von Mitteln zur Körperhygiene beim Menschen und beim Tier. Dies betrifft äußere Körperoberflächen und Strukturen wie die Haut und Hautfortsätze (beispielsweise Haare, Borsten, Federn, Nägel, Klauen oder Schnäbel) sowie innere Oberflächen und Strukturen wie zum Beispiel die Mundhöhle (das Maul) mit den Zähnen und den Rachen. In Hygienemitteln enthaltende antimikrobielle Wirkstoffe sind zum Beispiel Schwefelverbindungen (beispielsweise Methyl(chlor)isothiazolinon), Säuren (beispielsweise Benzoesäure, Sorbinsäure, Ameisensäure und Salicylsäure), organische Alkohole (beispielsweise Farnesol), Phenole (beispielsweise Triclosan), Chlorverbindungen (beispielsweise Chlorhexidin), Metallverbindungen (beispielsweise Kupfersulfat) und Aldehyde (beispielsweise Formaldehyd).

Antimikrobiell wirksame Substanzen, wie sie im Rahmen hygienischer Maßnahmen oder auch gesundheitlicher Anwendungen eingesetzt werden, können auch in Form von Nanopartikeln oder auf der Oberfläche geeigneter Trägermaterialien immobilisiert als sogenannte Mikropartikel (Microbeads, Microspheres) formuliert sein. Nanopartikel haben typischerweise Durchmesser von bis zu 100 nm. Mikropartikel können Durchmesser bis zu einigen Mikrometern aufweisen. Sowohl Nanopartikel als auch Mikropartikel werden durch besondere, teils aufwändige chemisch-biochemisch-physikalische Verfahren hergestellt.

Die oben beschriebenen Mittel und Verfahren zur Desinfektion und Konservierung sowie Körperhygiene nach dem Stand der Technik beinhalten, gleich welcher Formulierung, ebenfalls verschiedene Nachteile. So sind viele desinfizierende Substanzen toxisch und gesundheitsschädlich oder stark aggressiv gegenüber Materialien und benötigen in Handhabung und Anwendung besondere Sicherheitsvorkehrungen. In Abwässer gelangende Desinfektions- und Konservierungsmittel, beziehungsweise die entsprechenden Wirkstoffe, können die Umwelt stark belasten. Grundsätzlich sind alle chemischen und biochemischen Wirkstoffe bedenklich, da sie, insbesondere im Rahmen der Körperhygiene potenziell über die Haut oder die Schleimhäute in den Körper aufgenommen werden können. Gesundheitliche Langzeitrisiken durch die Verwendung von Nanopartikeln als besondere Art der Formulierung sind noch nicht abzusehen, da Nanopartikel wie auch Feinstaub in Zellen eindringen und physiologische Veränderungen hervorrufen können.

Es besteht deshalb ebenfalls großer Bedarf an neuen und alternativen Mitteln zur Verbesserung der Hygiene.

### Beschreibung

Dies vorausgeschickt, liegt der vorliegenden Erfindung die Aufgabe zugrunde, eine neuartige Verwendung eines Mittels zur Verfügung zu stellen, das 1) als Futtermittelzusatzstoff, beziehungsweise Lebensmittelzusatzstoff die Darmflora bei Wirbeltieren und beim Menschen dadurch stabilisiert, dass es auf gram-positive Bakterien bakterizid wirkt. Dadurch ist es möglich, die Balance zwischen gram-positiven und gram-negativen Bakterien innerhalb der bakteriellen Darmflora und insbesondere auch die Anzahl risikobehafteter oder pathogener gram-positiver Bakterien im Darm zu modulieren, wobei die Nachteile der oben genannten alternativen Mittel zur Stabilisierung der Darmflora nach dem Stand der Technik vermieden werden und das 2) als Desinfektionsmittel zur Verbesserung des hygienischen Zustands fester Materialien oder Flüssigkeiten sowie als Konservierungsmittel zur Konservierung von festen und flüssigen Futter- und Nahrungsmitteln sowie anderen Mitteln wie beispielsweise Kosmetika, und ferner zur Körperhygiene, also insgesamt im Rahmen von Maßnahmen zur Verbesserung der Hygiene eingesetzt werden kann, wobei auch hier die Nachteile der oben Mittel zur Desinfektion, Konservierung und Körperhygiene nach dem Stand der Technik vermieden werden.

Die Erfindung betrifft verschiedene Verwendungen, die in den Ansprüchen 1, 2, 3 und 4 beschrieben sind. Vorteilhafte Ausführungsformen der Erfindung sind in den Unteransprüchen angegeben.

Das erfindungsgemäße Mittel enthält Sporen des Pilzes *Myceliophthora thermophila.* Das Mittel ist eine bakterizid wirksame Substanz und wird in dieser Anmeldung auch als "bakterizides Mittel" oder "Bakterizid" bezeichnet.

*Myceliophthora thermophila* ist ein thermophiler, filamentöser Pilz aus der Abteilung der *Ascomyceten,* der Klasse der *Sordariomycetes,* der Ordnung der *Sordariales* und der Familie der *Chaetomiaceae. Myceliophtora thermophila* wurde insbesondere von Apinis (Nova Hedwigia 5:57-78, 1963) und von van Oortschot (Persoonia 9:401-408, 1977) beschrieben. *Myceliophthora thermophila* kommt unter anderem in Komposten, Heu und warmen Böden vor, von wo er isoliert werden kann. Die vegetativen Sporen entwickeln sich einzeln oder in kurzen Reihen aus ampullenförmigen Verdickungen konidialer Hyphen heraus und haben einen Durchmesser von 3-5 µm. Im reiferen Stadium besitzen sie eine raue Oberfläche. Früher verwendete Synonyme für *Myceliophthora thermophila* sind *Chrysosporium thermophilum* und *Sporotrichum thermophile.* Die Teleomorphe (das sexuelle Stadium) trägt den Namen *Thielavia heterothallica* (früher *Corynascus heterothallicus*). Industriell wird *Myceliophthora thermophila* vor allem für die Produktion technischer Enzyme zum Abbau pflanzlicher Biomasse verwendet.
Der Pilz ist der Öffentlichkeit zugänglich, insbesondere durch Hinterlegungen von Stämmen bei anerkannten Hinterlegungsstellen, z.B. bei der American Type Culture Collection (ATCC) unter der Hinterlegungsnummer ATCC 42464, beim Westerdijk Fungal Biodiversity Institute unter der Hinterlegungsnummer CBS 117.65 und bei der Deutsche Sammlung von Mikroorganismen und Zellkulturen (DSMZ) unter der Hinterlegungsnummer DSM1807.
Der Stamm des Pilzes *Myceliophtora thermophila,* der für die Untersuchungen verwendet wurde, die in den Ausführungsbeispielen im Einzelnen angegeben sind, wurde mit der Identifikationsnummer Myceliphtora thermophila #154 hinterlegt beim Westerdijk Fungal Biodiversity Institute, CBS Collection, Uppsalaan 8, 3508 AD Utrecht. Die Hinterlegungsstelle hat für die Hinterlegung die Eingangsnummer CBS 146310 vergeben.

Es wurde überraschend festgestellt, dass Sporen des Pilzes *Myceliophthora thermophila,* die von einer Kultur dieses Pilzes stammen, auf gram-positive Bakterien bakterizid wirken, ohne dass es darüber hinaus einer besonderen Behandlung der Sporen bedarf. Dies haben Vergleichsuntersuchungen ergeben, bei denen die Wirkung einer Sporensuspension von *Myceliophthora thermophila* auf eine Auswahl gram-positiver und gram-negativer Bakterien untersucht wurde und zudem getestet wurde, ob die Wirkung bakteriostatischer oder bakterizider Natur ist. Die anti-bakterielle Wirkung wurde in drei verschiedenen Testverfahren unabhängig voneinander nachgewiesen: 1) Beim "PREMI-Test" wird die potenzielle antibakterielle Wirkung innerhalb eines Nähragars dadurch geprüft, dass das Auskeimen von Sporen und das Auswachsen zu vegetativen Zellen des Testbakteriums *Bacillus stearothermophilus* nach der Zugabe einer Flüssigkeit und der Testsubstanz durch den Farbumschlag einer im Zuge dieser Vorgänge gebildeten Säure gemessen wird. 2) Beim "4-Platten-Test" wird die antibakterielle Wirkung durch die Abnahme der Trübung eines Nähragars geprüft, in den das jeweilige Testbakterien eingegossen wurde, wobei die Testsubstanz in ein ausgestanztes Loch im Agar gegeben wird. 3) Beim Bakterienwachstumstest in flüssigem Nährmedium wird die mit dem Wachstum des jeweiligen Testbakteriums zunehmende optische Dichte der Bakteriensuspension nach Zugabe der Testsubstanz photometrisch gemessen. In allen drei Testverfahren zeigte eine Sporensuspension von *Myceliophthora thermophila* antibakterielle Effekte gegenüber gram-positiven Bakterien. Hingegen zeigte die Sporensuspension gegenüber dem gram-negativen Bakterium *Escherichia coli* im 4-Platten-Test und im Bakterienwachstumstest mit flüssigem Nährmedium keinen antibakteriellen Effekt.

Dass es die Sporen von *Myceliophthora thermophila* sind, die dem bakteriziden Effekt gegenüber gram-positiven Bakterien als Wirkprinzip zugrunde liegen, wurde in einer weiteren Untersuchung gezeigt, bei der die Sporen durch Filtration aus der Sporensuspension entfernt wurden. Die verbleibende Lösung ohne Sporen zeigte keine bakterizide Wirkung. Deshalb ist davon auszugehen, dass es sich bei dem zugrundeliegenden Wirkprinzip nicht um eine wasserlösliche Substanz handelt, die entweder konstitutiv im Cytoplasma der lebenden Sporen gebildet und durch die Sporenwand in die umgebende Lösung abgegeben wird oder die sich auf der Oberfläche der Sporen als deren konstitutiver Bestandteil befindet und von dort ohne weiteres in die Lösung gelangt.

Weitere Untersuchungen haben gezeigt, dass die bakterizide Wirkung gegenüber grampositiven Bakterien auch nicht-lebenden, inaktivierten Sporen zukommt. Dazu wurden die Sporen durch eine Säuremischung (Schaumacid^{®} der H. Wilhelm Schaumann GmbH & Co KG) abgetötet, sodass sie nicht mehr in der Lage waren, auszukeimen. Auch dies spricht eindeutig gegen eine von den Sporen im aktiven, cytoplasmatischen Stoffwechsel gebildete und dann sezernierte bakterizide Substanz als Wirkprinzip. Zudem blieb die bakterizide Wirkung unvermindert erhalten, wenn die Sporen getrocknet und danach erneut suspendiert wurden. Ebenfalls blieb die bakterizide Wirkung erhalten, wenn die Sporen zur Auskeimung gebracht und von dem sich entwickelnden Pilzmycel im weiteren Verlauf gebildete Sporen geerntet und auf ihre antibakterielle Wirkung hin getestet wurden. Die bakterizide Wirkung der Sporen ist folglich generationstabil.

Hitzebehandelte Sporen (durch Autoklavieren) von *Myceliophthora thermophila* zeigten gegenüber nicht hitzebehandelten Sporen einen stark verminderten bakteriziden Effekt.

Mit beginnender Keimung ging die bakterizide Wirkung der Sporen von *Myceliophthora thermophila* verloren.

Die bakterizide Wirkung der Sporen von *Myceliophthora thermophila* gegenüber gram-positiven Bakterien zeigte sich insbesondere dann, wenn die Sporen durch Kultivierung des Pilzes auf Kartoffel-Dextrose-Agar oder auf Vermiculit zusammen mit Kartoffelinfus und Glucose hergestellt worden waren.

Sporen der nahe verwandten Art *Myceliophthora lutea* zeigten unter sonst gleichen Bedingungen keinen antibakteriellen Effekt gegenüber gram-positiven Bakterien. Auch Sporen anderer Arten filamentöser Pilze, beispielsweise von Stämmen der Arten *Aspergillus oryzae, Neurospora intermedia* und *Chaetomium thermophilum* (zur gleichen Familie wie *Myceliophthora thermophila* gehörend) zeigten keine antibakterielle Wirkung gegenüber gram-positiven Bakterien. Es ist deshalb davon auszugehen, dass die antibakterielle Wirkung gegenüber gram-positiven Bakterien keine allgemeine Eigenschaft von Sporen filamentöser Pilze oder überhaupt von Pilsporen ist.

Einzelheiten zu den beschriebenen Untersuchungen sind in den Ausführungsbeispielen angegeben.

Der Mechanimus, welcher der bakteriziden Wirkung der Sporen von *Myceliophthora thermophila* zugrunde liegt, konnte bisher nicht im Detail aufgeklärt werden. Die Ergebnisse der bisherigen Untersuchungen legen aber folgende, charakteristische Eigenschaften des Wirkprinzips nahe, die als Merkmale der Erfindung gelten können: 1) Bei dem, dem beobachteten bakteriziden Effekt zugrundliegenden Wirkprinzip handelt es sich um eine Eigenschaft der Sporenoberfläche, das heißt, die Sporenoberfläche ist in diesem Sinne "bio-aktiv". Zur Entfaltung des bakteriziden Effekts bedarf es entweder eines direkten Kontakts der Bakterienzellen mit der Sporenoberfläche oder einer den Effekt vermittelnden Substanz, welche entweder eine Signalsubstanz ist, die in Kontakt mit der Sporenoberfläche treten muss, oder eine Vorstufe einer bakteriziden Substanz, welche durch ein Enzym (oder mehrere Enzyme), das an die Sporenoberfläche gebunden ist, in die bakterizide Substanz umgewandelt wird. In beiden Fällen kann die den Effekt vermittelnde Substanz nicht aus der in den oben beschriebenen Untersuchungen verwendeten Sporensuspension stammen. 2) Zugleich ist der bakterizide Effekt eindeutig nicht auf die unmittelbare Umgebung der Sporen beschränkt, das heißt, er breitet sich, einmal initiiert, räumlich aus und die bakterizide Wirkung entfaltet sich auch in einer gewissen Distanz vom Ort der Sporen. Dies zeigen die Untersuchungen der bakteriziden Wirkung der Sporen von *Myceliophthora thermophila* in Testverfahren, bei denen feste Nährmedien verwendet wurden, in denen weder die Testbakterien, noch die Pilzsporen aufgrund ihrer jeweiligen Größen beweglich sind. 3) Es ist zwar denkbar, aber unwahrscheinlich und nicht plausibel anzunehmen, dass es sich bei der - sofern das Signal zur Initiierung der bakteriziden Wirkung nicht der physische Kontakt der Bakterienzellen mit der Sporenoberfläche ist - postulierten Signalsubstanz bzw. Vorstufe um Substanzen handelt, die ohne weiteres in den, bei den oben beschriebenen Testverfahren verwendeten Nährmedien unter den in den einzelnen Untersuchungen variierenden Bedingungen vorkommen. Wahrscheinlicher ist, und dies wird hier postuliert, dass es sich dabei um Substanzen handelt, die in Verbindung mit der Anwesenheit gram-positiver Bakterien in den jeweiligen Nährmedien stehen bzw. durch die Bakterien selbst generiert werden, so dass man annehmen kann, dass der, der bakteriziden Wirkung zugrunde liegende Mechanismus die Anwesenheit der gram-positiven Bakterien "erkennt".

Das erfindungsgemäße Mittel weist diejenigen Vorteile auf, wie sie in Form von auf Mikropartikeln immobilisierten Wirkstoffen durch diese besondere Art der Formulierung zukommen. Dazu gehört generell eine verbesserte Stabilität. Gegenüber Nanopartikeln weisen die Pilzsporen den Vorteil auf, aufgrund ihrer Größe nicht in Zellen des Darmepithels eindringen können. Ein Vorteil gegenüber mit antimikrobiellen Wirkstoffen beschichteten Mikropartikeln, wie sie nach dem Stand der Technik zur Verfügung stehen und bei denen die bakterizide Wirkung einen direkten Kontakt der Bakterienzellen mit der Oberfläche der Partikel voraussetzt, ist die Reichweite des bakteriziden Effekts des erfindungsgemäßen Mittels über die unmittelbare Region an der Sporenoberfläche hinaus, so dass hier nicht jede Bakterienzelle mindestens einmal mit der Oberfläche mindestens eines Mikropartikels in Berührung gekommen sein muss, um abgetötet zu werden. In dem Falle, in dem Mikropartikel nach dem Stand der Technik mit einem auf Distanz wirkenden, bakteriziden Mechanismen ausgestattet sind, ist bei dem hier beschriebenen, erfindungsgemäßen Mittel der besondere Vorteil, dass es nur dann seine bakterizide Wirkung entfaltet, wenn die Pilzsporen in Reichweite gram-positiver Bakterien sind, das heißt die bakterizide Wirkung entfaltet sich nicht ohne weiteres, sondern bei Bedarf. Ein weiterer praktischer Vorteil des erfindungsgemäßen Mittels gegenüber der Verwendung antimikrobiell wirkender Mikropartikel nach dem Stand der Technik ist, dass die Sporen von *Myceliophthora thermophila* die bakterizide Wirkung nach Kultivierung des Pilzes zeigen. Darüber hinaus bedarf es keiner weiteren notwendigen und aufwändigen Beschichtung der Sporen oder sonstiger komplexer chemisch-biochemischphysikalischer Behandlungen. Die Sporen schädigen zudem nicht die Umwelt, da Sporen von *Myceliophthora thermophila* ubiquitär unter natürlichen Bedingungen vorkommen.

Zusammenfassend wird deshalb davon ausgegangen, dass die hier beschriebene, bakterizide Wirkung von Sporen von *Myceliophthora thermophila* mit ihren besonderen, wirkmechanistischen Eigenschaften eine völlig neue Entdeckung ist. Ein vergleichbares Mittel, das heißt ein Mittel mit identischen Wirkeigenschaften auf Basis von Mikropartikeln mit einer geeigneten Beschichtung oder sonstigen Behandlung synthetisch herzustellen, würde äußerst komplexe Bearbeitungsschritte und molekulare Ausstattungen der Mikropartikel erfordern. Die erfindungsgemäßen Eigenschaften der Sporen von *Myceliophthora thermophila* sind zudem nicht naheliegend, da eine bakterizide Wirkung gegenüber gram-positiven Bakterien nicht zu den bekannten und erwartbaren Eigenschaften von Pilzsporen im Allgemeinen gehört. Mit dem hier beschriebenen, neu gewonnenen Wissen kann künftig jedoch nicht grundsätzlich ausgeschlossen werden, dass auch unter anderen, als den oben genannten Herstellungsbedingungen und auch bei Sporen anderer Pilzarten antimikrobielle Effekte mit den hier beschriebenen, besonderen erfindungsgemäßen Merkmalen auftreten können.

US 2002/0102246 A1 beschreibt eine enzymatische Zusammensetzung, die sowohl für die Desinfektion in Wäschereien, auf festen Oberflächen, in Wassersystemen, auf der Haut, auf den Zähnen oder auf Schleimhäuten als auch für die Konservierung von Nahrungsmittelprodukten, Kosmetika, Farben und Beschichtungen verwendet werden kann. Diese umfasst ein Phenol-oxidierendes Enzymsystem und einen Enhancer. Das Phenol-oxidierende Enzymsystem kann zum Beispiel eine Laccase oder ein mit Laccase verwandtes Enzym zusammen mit Sauerstoff oder einem anderen Oxidationsmittel sein. In der Patentschrift wird besonders auf solche Laccasen fokussiert, die von Pilzen gebildet werden. Zu den Laccase-bildenden Pilzen gehört unter anderem auch *Myceliophthora thermophila.* Eine Laccase als Bestandteil des Mechanismus der bakteriziden Wirkung der Sporen von *Myceliophthora thermophila* konnte jedoch experimentell nicht nachgewiesen werden. Zudem wurde in einer externen Studie (siehe die Anlage) gezeigt, dass die Sporen von *Myceliophthora thermophila* auch unter anaeroben (sauerstofffreien) und reduzierenden Bedingungen bakterizid gegen anaerobe, gram-positive Bakterien wirken. Dies schließt eine Laccase sowie auch andere Oxidasen als am Wirkmechanismus Beteiligte aus.

US 7.288.264 B1 beschreibt verschiedene antimikrobiell wirkende Gegenstände bzw. Mittel, die Mikroorganismen bei Kontakt abtöten, darunter auch Mikropartikel. Die Erfindung beschreibt jedoch keine Gegenstände bzw. Mittel, bei denen der antimikrobielle Effekt nicht auf die unmittelbare Oberfläche der Gegenstände beschränkt ist.

WO 2011/033275 Al beschreibt Bakteriensporen, die an ihrer Oberfläche mit einem therapeutisch wirksamen Mittel ausgestattet sind. Auch diese Erfindung beschreibt keine mikrobiellen Systeme, bei denen der antimikrobielle Effekt nicht auf die unmittelbare Oberfläche der Sporen beschränkt ist.

US 2006/0247 150 A1 beschreibt Zusammensetzungen für desinfizierende und Reinigungsprozesse, die Sporen von Pilzen enthalten. Die Sporen tragen jedoch gemäß dieser Erfindung keine bakterizide Wirkung.

Das erfindungsgemäße Mittel kann zur Stabilisierung der Darmflora oder der Bakterienflora von Hautoberflächen, Körperöffnungen, Mund (bzw. Maul), Rachen oder anderen äußeren oder inneren Körperoberflächen bei Wirbeltieren und beim Menschen eingesetzt werden, sofern es inaktivierte Sporen von *Myceliophthora thermophila* enthält. Es wird erwartet, dass das Mittel die Zusammensetzung der Darmflora oder einer anderen Bakterienflora durch Modulation der Anzahl gram-positiver Bakterien positiv beeinflusst und die Widerstandsfähigkeit gegenüber gram-positiven, krankheitsverursachenden Bakterien erhöht. Letzteres ist im Falle der Darmflora besonders während sensibler Stadien im Rahmen der Aufzucht beziehungsweise des Wachstums von großer Bedeutung und reduziert das Risiko von Durchfällen und anderen bakteriellen Erkrankungen. Insgesamt wird erwartet, dass sich das Wohlbefinden von Tieren und des Menschen durch die Anwendung des Mittels deutlich verbessert. Darüber hinaus kann das Mittel in therapeutischer Dosierung zur Vorbeugung und Behandlung von Erkrankungen beim Menschen und bei Wirbeltieren eingesetzt werden.

Ferner kann das Mittel zur Verbesserung der Hygiene, das heißt als Desinfektionsmittel und als Konservierungsmittel für die Behandlung fester und flüssiger Materialien sowie für die Körperhygiene von Mensch und Tier eingesetzt werden mit dem Ziel, gram-positive Bakterien abzutöten oder dem Wachstum gram-positiver Bakterien vorzubeugen.

### Ausführungsformen

Gemäß einer Ausführungsform enthält das erfindungsgemäße Mittel von einer Kultur des Pilzes *Myceliophtora thermophila* stammende Sporen dieses Pilzes. Gemäß einer weiteren Ausführungsform enthält das Mittel von einer Kultur des Pilzes *Myceliphthora thermophila* isolierte Sporen dieses Pilzes. Gemäß einer weiteren Ausführungsform werden die Sporen von der Kultur des Pilzes isoliert, indem sie von der Kultur geerntet werden. Gemäß einer weiteren Ausführungsform werden die Sporen wiederholt von der Kultur geerntet. Gemäß einer weiteren Ausführungsform werden Sporen mitsamt der Kultur oder Teilen der Kultur als bakterizides Mittel verwendet. Gemäß einer weiteren Ausführungsform enthält das erfindungsgemäße Mittel inaktivierte, das heißt abgetötete Sporen des Pilzes *Myceliophthora thermophila.*

Gemäß einer Variante dieser Ausführungsformen enthält das erfindungsgemäße Mittel durch eine oder mehrere futtermittelrechtlich, nahrungsmittelrechtlich oder gemäß der EU-Biozid-Verordnung sowie anderer relevanter Verordnungen und Gesetze zugelassene Säuren, im Falle der Verwendung als Futtermittelzusatzstoff beispielsweise durch das Produkt Schaumacid^{®} der H. Wilhelm Schaumann GmbH & Co KG, Ovelgünner Straße 27, 39365 Eilsleben, abgetötete Sporen. Die Behandlung mit Säuren kann für die Herstellung des erfindungsgemäßen Mittels eingesetzt werden.

Gemäß einer weiteren Ausführungsform ist das erfindungsgemäße Mittel ein Feststoff, der Sporen von *Myceliophthora thermophila* enthält. Eine Formulierung als Feststoff ist dann besonders vorteilhaft, wenn das Mittel in andere Feststoffe eingemischt werden soll, ohne dass dabei zusätzliche Feuchtigkeit in die Zubereitung eingetragen werden soll.

Gemäß einer Variante dieser Ausführungsform werden die Sporen von der Pilzkultur trocken geerntet, indem sie beispielsweise von der Pilzkultur abgesaugt und anschließend gesammelt oder indem sie mit Hilfe von adhäsiv wirkenden Substanzen, wie beispielsweise Saccharose oder Lactose von der Pilzkultur aufgenommen und die adhäsive Substanz zusammen mit den adhärierten Sporen von dem Restmaterial der Pilzkultur durch Sieben abgetrennt wird. Hierdurch entsteht eine feste Sporenformulierung auf einem Trägermaterial.

Gemäß einer weiteren Variante dieser Ausführungsform werden die Sporen von der Pilzkultur mittels einer Salinelösung (einer salzhaltigen, wässrigen Lösung, wobei das Salz zum Beispiel Kochsalz ist) aufgenommen und die Sporensuspension anschließend getrocknet. Dabei kann die Salinelösung zusätzliche Stoffe enthalten, welche die Suspendierung der Sporen in der Salinelösung fördern, zum Beispiel oberflächenaktive Stoffe wie Tween 20 oder Tween 80 oder andere Tenside.

Gemäß einer Variante dieser Ausführungsform wird die Sporensuspension durch Gefriertrocknung, Vakuumtrocknung oder Sprühstrocknung getrocknet. Die Gefriertrocknung, Vakuumtrocknung oder Sprühtrocknung sind besonders schonende Verfahren der Trocknung und geeignet, die Haltbarkeit der Sporen zu erhöhen.

Gemäß einer weiteren Variante dieser Ausführungsform werden der festen Sporenformulierung weitere Stoffe zugegeben, welche die physikalischen Eigenschaften und die weitere Verarbeitung, zum Beispiel das Einmischen in feste oder flüssige Zubereitungen, verbessern oder welche die Haltbarkeit erhöhen. Dies können Trägerstoffe oder Bindemittel sein, wie beispielsweise Salze der Essigsäure oder Bentonit, Fließmittel wie beispielsweise Kieselgur, Konservierungsmittel wie beispielsweise organische Säuren und Tenside wie beispielsweise Tween 20 oder Tween 80 sowie andere Stoffe.

Gemäß einer weiteren Ausführungsform wird das erfindungsgemäße Mittel in Form einer Paste oder einer Creme zur Verfügung gestellt, welche die Sporen von *Myceliophthora thermophila* enthält. Formulierungen des Mittels in Form einer Paste oder Creme sind besonders für eine desinfizierende Behandlung von Oberflächen fester Materialien oder zur Körperhygiene geeignet.

Gemäß einer weiteren Ausführungsform wird das erfindungsgemäße Mittel in Form einer flüssigen Zubereitung, welche die Sporen von *Myceliophthora thermophila* enthält, zur Verfügung gestellt. Flüssige Formulierungen des Mittels sind ebenfalls unter anderem für die desinfizierende Behandlung von Oberflächen fester Materialien oder äußerer und innerer Körperoberflächen sehr gut geeignet.

Gemäß einer Variante dieser Ausführungsform basiert die flüssige Formulierung auf Wasser oder Alkohol.

Gemäß einer weiteren Variante dieser Ausführungsform enthält die flüssige Formulierung auf Basis von Wasser oder Alkohol neben den Sporen von *Myceliophthora thermophila* weitere Stoffe oder Substanzen, welche die physikalischen Eigenschaften und die weitere Verarbeitung verbessern oder welche die Haltbarkeit der flüssigen Formulierung erhöhen. Dabei kann es sich um Stoffe handeln, welche die Suspendierung der Sporen in der Flüssigkeit aufrechterhalten.

Gemäß einer weiteren Ausführungsform, wird das erfindungsgemäße Mittel als Futtermittelzusatzstoff bzw. Nahrungsmittelzusatzstoff zur Stabilisierung der Darmflora von Wirbeltieren, insbesondere von Säugetieren und Vögeln, insbesondere von Nutztieren (wie beispielsweise Rindern, Pferden, Schafen, Ziegen, Schweinen und Geflügel) und Haustieren (wie beispielsweise Hunden und Katzen) und des Menschen durch Modulation der Anzahl gram-positiver Bakterien eingesetzt. Beim Einsatz des erfindungsgemäßen Mittels bei Nutztieren in der Landwirtschaft ist eine Steigerung der Leistungsparameter während des Wachstums der Tiere wie beispielsweise der mittleren täglichen Lebendmassezunahme und der Futtereffizienz (der pro Einheit Lebendmassezunahme aufgenommenen Futtermenge) zu erwarten. Ferner ist über alle Lebensstadien hinweg eine erhöhte Widerstandskraft gegenüber negativen Effekten gram-positiver Bakterien zu erwarten. Besondere Bedeutung hat dies während kritischer Stadien im Verlauf der Aufzucht, beispielsweise bei Ferkeln während der Absetzphase. Insgesamt trägt der Einsatz des erfindungsgemäßen Mittels, insbesondere in der intensiven landwirtschaftlichen Nutztierhaltung zur Steigerung des Tierwohls bei. In einer Untersuchung (Ergebnisse nicht dargestellt) konnte gezeigt werden, dass der bakterizide Effekt der Sporen von *Myceliophthora thermophila* stabil gegenüber der Behandlung mit einer Protease ist, sodass davon ausgegangen werden kann, dass die bakterizide Wirkung der Sporen gegenüber gram-positiven Bakterien nach der Passage des Magens und im Darm erhalten bleibt. Die Pilzsporen führten in einer, gemäß der offiziell festgelegten Methode O.E.C.D. Test Guideline No. 423 vom 17. Dezember 2001 durchgeführten Studie nicht zu klinischen Krankheitsanzeichen oder zur Mortalität. Der LD 50 liegt über 5000 mg/kg Körpergewicht bei oraler Aufnahme durch Ratten. Die Pilzsporen werden mit den Exkrementen wieder ausgeschieden. Untersuchungen haben gezeigt, dass die Pilzsporen methanbildende Bakterien nicht inhibieren, sodass die Exkremente der Biogaserzeugung zugeführt werden können.

Gemäß einer Variante dieser Ausführungsform, wird das erfindungsgemäße Mittel dazu in fester Form in Futtermittel oder Nahrungsmittel eingemischt.

Gemäß einer weiteren Variante dieser Ausführungsform, wird das erfindungsgemäße Mittel in fester oder flüssiger Form in Tränkewasser und Trinkwasser beziehungsweise beim Menschen in andere Getränke (wie beispielsweise in Obst- oder Gemüsesäfte) gegeben.

Gemäß einer weiteren Variante dieser Ausführungsformen wird die Dosierungsempfehlung anhand der Ergebnisse einer Mikrobiomanalyse, das ist eine Analyse der Zusammensetzung der Darmflora, getroffen, insbesondere anhand einer Bestimmung des Verhältnisses der Anzahlen von grampositiven zu gram-negativen Bakterien, insbesondere durch Bestimmung der Anzahlen risikobehafteter gram-positiver Bakterien.

Gemäß einer weiteren Ausführungsform werden die Sporen von *Myceliophthora thermophila* in therapeutischer Dosierung zur Behandlung und Vorbeugung bakterieller Erkrankungen, insbesondere von Darmerkrankungen, sowie anderer Erkrankungen wie Entzündungen der Schleimheute beim Menschen und bei Wirbeltieren eingesetzt.

Gemäß einer weiteren Ausführungsform werden die Sporen von *Myceliophthora thermophila* in fester oder flüssiger Formulierung zur Desinfektion fester Materialen oder von Oberflächen fester Materialien oder von Flüssigkeiten im Kontaktbereich von Tieren und Menschen mit dem Ziel eingesetzt, dort gram-positive Bakterien abzutöten.

Gemäß einer Variante dieser Ausführungsform werden die Sporen von *Myceliophthora thermophila* als Bestandteil der Einstreu in Stallungen im Rahmen der Haltung von Nutztieren oder Haustieren eingesetzt.

Gemäß einer Variante dieser Ausführungsform wird eine wässrige Formulierung der Sporen oder eine Formulierung in Alkohol als Desinfektionsmittel verwendet.

Gemäß einer weiteren Variante dieser Ausführungsform enthält die wässrige Formulierung oder die Formulierung in Alholol weitere desinfizierend wirkende Substanzen, beispielsweise organische Säuren oder Tenside.

Gemäß einer weiteren Variante dieser Ausführungsform wird eine pastöse oder gelartige Formulierung der Sporen verwendet, die auf die Oberflächen fester Materialien aufgestrichen wird und nach einer geeigneten Einwirkzeit abgewaschen werden kann.

Gemäß einer weiteren Ausführungsform wird das erfindungsgemäße Mittel zur Konservierung von festen und flüssigen Materialien eingesetzt. Damit soll das Wachstum grampositiver Bakterien in diesen Materialien unterdrückt werden.

Gemäß einer Variante dieser Ausführungsform wird das erfindungsgemäße Mittel zusammen mit anderen Konservierungsmitteln, beispielsweise organischen Säuren eingesetzt.

Gemäß einer weiteren Ausführungsform wird das erfindungsgemäße Mittel zur Körperhygiene bei Menschen und Tieren eingesetzt.

Gemäß einer Variante dieser Ausführungsform wird das erfindungsgemäße Mittel zum Beispiel festen Seifen, Waschlotions, Schampoos, Zahnpasten oder Deodorants zur Pflege von äußeren und inneren Körperoberflächen beim Menschen und bei Wirbeltieren eingesetzt. Insbesondere kann man sich auch bei Hygienemitteln die abrasiven Eigenschaften, die durch die Größe und Oberflächenbeschaffenheit der Sporen bestimmt werden, für Reinigungszwecke zunutze machen.

Gemäß einer weiteren Variante dieser Ausführungsform wird das erfindungsgemäße Mittel bei der Klauenreinigung oder als Bestandteil von Euter-Dips bei Nutztieren eingesetzt.

Gemäß einer weiteren Ausführungsform wird das erfindungsgemäße Mittel zum Abtöten der folgenden gram-positiven Bakterien verwendet: *Bacillus subtilis, Bacillus stearothermophilus, Bacillus cereus, Staphylococcus aureus, Enterococcus faecalis, Clostridium perfringens* und *Clostridium tyrobutyricum.*

Die Erfindung wird nachfolgend anhand der Beschreibung von Ausführungsbeispielen unter Bezug auf die anliegenden Abbildungen erläutert.

### Ausführungsbeispiele

Im Folgenden werden unter Bezug auf die beigefügten Abbildungen Ausführungsbeispiele vorgestellt, ohne dass die Erfindung auf diese Beispiele beschränkt ist. Die Abbildungen zeigen:
Abbildung 1:
   Nachweis der antibakteriellen Wirkung von Sporensuspensionen von *Chaetomium thermophilum* und *Myceliophthora thermophila* mit dem PREMI-Test.
Abbildung 2:
   Wirkung verschiedener Sporensuspensionen auf *Bacillus subtilis.*
Abbildung 3:
   Nachweis der antibakteriellen Wirkung von Sporen von *Myceliophthora thermophila,* wobei der Pilz auf unterschiedlichen Wachstumssubstraten kultiviert wurde, mit dem PREMI-Test.
Abbildung 4:
   Nachweis der antibakteriellen Wirkung autoklavierter und nicht-autoklavierter Sporensuspension von *Myceliophthora thermophila* auf gram-positive Bakterien mit dem 4-Platten-Test.
Abbildung 5:
   Bestimmung der minimalen Hemmkonzentration der Sporensuspension von *Myceliophthora thermophila* gegen *Bacillus subtilis.*
Abbildung 6:
   Nachweis der antibakteriellen Wirkung der Sporensuspension von *Myceliaphthora thermophila* auf *Escherichia coli.*
Abbildung 7:
   Wirkung der Sporensuspension von *Myceliophthora thermophila* auf *Escherichia coli.*
Abbildung 8:
   Wirkung von Cycloheximid auf *Bacillus subtilis* und Sporen von *Myceliophthora thermophila.*
Abbildung 9:
   Untersuchung der bakteriziden beziehungsweise bakteriostatischen Wirkung der Sporensuspension von *Myceliophthora thermophila.*
Abbildung 10:
   Wirkung inaktivierter Sporensuspension von *Myceliophthora thermophila* auf *Bacillus subtilis.*
Abbildung 11:
   Wirkung der Sporensuspension von *Myceliophthora thermophila* mit 2% Schaumacid und einer Kontrolle aus Saline mit 2% Schaumacid auf *Bacillus subtilis.*
Abbildung 12:
   Wirkung von gefriergetrockneten Sporen von *Myceliophthora thermophila* auf *Bacillus subtilis.*
Abbildung 13:
   Wirkung der Sporen aus der dritten Generation von *Myceliophthora thermophila* auf *Bacillus subtilis* und *Kocuria rhizophila.*
Abbildung 14:
   Lagerstabilität der antibakteriellen Wirkung der Sporen von *Myceliophthora thermophila.*

### Nachweis der antibakteriellen Wirkung von Sporensuspensionen von Chaetomium thermophilum und Myceliophthora thermophila mit dem PREMI-Test

Bei dem PREMI-Test handelt es sich um einen einfachen mikrobiellen Screening-Test mit breitem Spektrum zum Nachweis antimikrobieller Substanzen, wie Antibiotika. Das Testprinzip basiert auf einer Wachstumshemmung von *Bacillus stearothermophilus.* Dazu wird eine standardisierte Anzahl Sporen von *Bacillus stearothermophilus* in einem Nährstoffagar eingebettet. Nach Zugabe einer Flüssigkeit keimen die Sporen aus und bilden eine Säure, die zu einem Farbumschlag des Nährstoffagars von Violett zu Gelb führt. Bei einer Wachstumshemmung durch einen ebenfalls zugesetzten antibakteriellen Wirkstoff kommt es zu keinem Farbumschlag. Ampicillin ist ein wirksames Antibiotikum gegen *Bacillus stearothermophilus* und dient im Test als Positivkontrolle. Als Negativkontrolle dient Wasser, da es hierdurch zu keiner Wachstumshemmung kommen sollte.

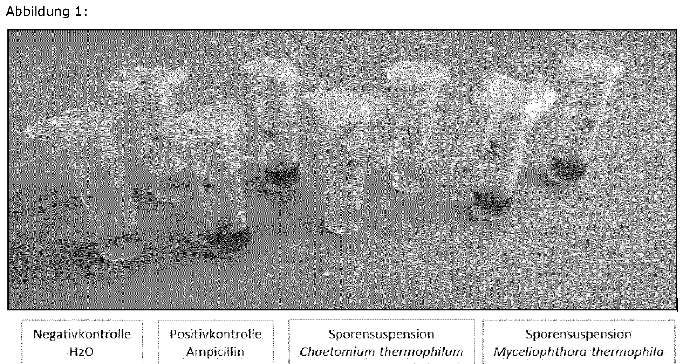

Die Ergebnisse (siehe Abbildung 1) zeigen, dass eine Sporensuspension von *Myceliophthora thermophila,* die von einer Kultur auf Kartoffel-Dextrose-Agar geerntet wurde, das Wachstum von *Bacillus stearothermophilus* unterdrücken konnte. Die Sporensuspension eines anderen thermophilen, filamentösen Pilzes, *Chaetomium thermophilum,* zeigte keine antibakterielle Wirkung gegenüber *Bacillus stearothermophilus.*

### Wirkung verschiedener Sporensuspensionen auf Bacillus subtilis

Bei dem Bakterienwachstumstest in flüssigem Nährmedium zum Nachweis antibakterieller Effekte von Wirkstoffen wird zunächst eine Vorkultur des jeweiligen Zielbakteriums hergestellt. Eine Sporensuspension mit einer definierten Anzahl Pilzsporen wird mit der Bakteriensuspension beimpft und bebrütet. Anhand des Verlaufs der Trübung (gemessen als OD₆₀₀) kann der Verlauf des Wachstums des Zielbakteriums gemessen werden. Die Konzentration von Sporen in der Sporensuspension, bei der gerade noch kein Bakterienwachstum stattfindet, ist die minimale Hemmstoffkonzentration (MHK).

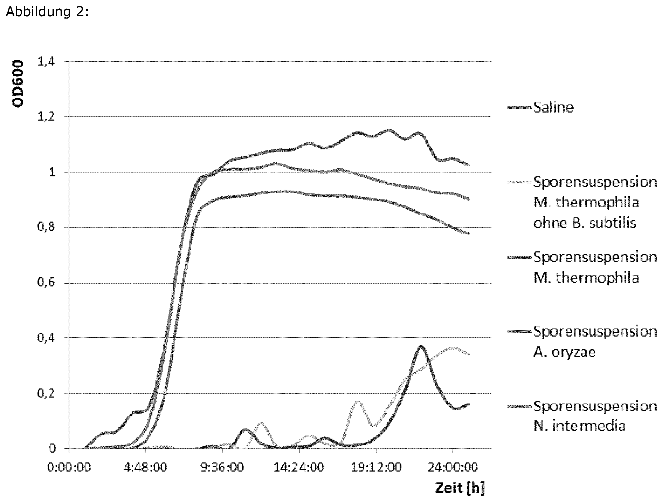

In einer ersten Untersuchung wurden Sporensuspensionen von *Myceliophthora thermophila, Aspergillus oryzae* und *Neurospora intermedia* und eine Bakteriensuspension mit *Bacillus subtilis* als Zielbakterium verwendet (siehe Abbildung 2). Als Kontrolle wurde eine Salinelösung zu der Bakteriensuspension zugegeben. In Anwesenheit der Salinelösung fand ein deutliches Wachstum von *Bacillus subtilis* statt. Die Sporensuspensionen von *Aspergillus oryzae* und *Neurospora intermedia* zeigten ebenfalls keine anti-bakterielle, wachstumshemmende Wirkung auf *Bacillus subtilis.* Dagegen zeigte die Sporensuspension von *Myceliophtora thermophila* eine deutliche hemmende Wirkung auf das Wachstum von *Bacillus subtilis.* Als Kontrolle wurde außerdem der Trübungsverlauf der Sporensuspension von *Myceliophthora thermophila* alleine, das heißt ohne Zulage der Bakteriensuspension photometrisch gemessen. Hierbei ist erkennbar, dass die Pilzsporen nach etwa 19 Stunden auskeimen. Folglich ist der Anstieg der Trübung nach etwa 19 h bei Einsatz der Sporensuspension von *Myceliophthora thermophila* zusammen mit der Suspension von *Bacillus subtilis* auf das Auskeimen der Sporen von *Myceliophthora thermophila,* nicht auf ein Wachstum von *Bacillus subtilis,* zurückzuführen.

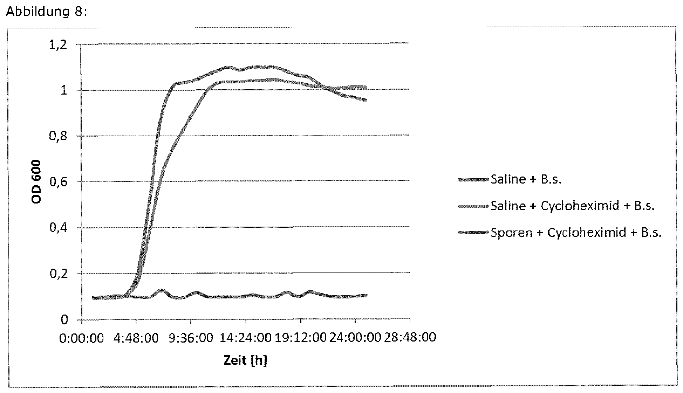

Die wachstumshemmende Wirkung der Sporensuspension von *Myceliophthora thermophila* auf *Bacillus subtilis* wurde in einer weiteren Untersuchung bestätigt (siehe Abbildung 8). Bei dieser Untersuchung wurde zu der Sporenlösung von *Myceliophthora thermophila* Cycloheximid zugegeben. Cycloheximid ist ein Translationshemmer, der zum Absterben eukaryontischer Zellen führt. Dieser Ansatz wurde verwendet, um zu zeigen, dass die antibakterielle Wirkung der Sporensuspension von *Myceliophthora thermophila* auf *Bacillus subtilis* auch über den Zeitraum von 19 h hinaus andauert. Als Kontrolle wurde Salinelösung mit der gleichen Konzentration an Cycloheximid zur Baktriensuspension zugegeben. Wie die Kontrolle zeigte, hatte das Cycloheximid selbst keinen Einfluss auf das Wachstum von *Bacillus subtilis.*

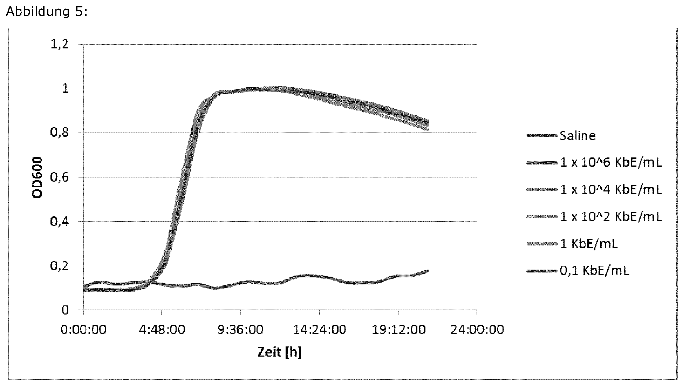

In einer weiteren Untersuchung wurde die Minimale Hemmkonzentration (MHK) der Sporensuspension von *Myceliophthora thermophila* auf das Wachstum von *Bacillus subtilis* näherungsweise bestimmt. Abbildung 5 zeigt, dass die MHK für *Bacillus subtilis* bei einer Sporenkonzentration zwischen 1·10⁴ KbE/mL und 1·10⁶ KbE/mL liegt (KbE = Koloniebildende Einheiten). Eine Sporenkonzentration in der Sporensuspension von 1·10⁶ KbE/mL führte klar zu einer vollständigen Hemmung des Wachstums von *Bacillus subtilis.* Diese Sporenkonzentration entspricht einem Verhältnis von 100 Pilzsporen pro Bakterienzelle. Nicht dargestellte Ergebnisse einer weiteren Untersuchung zeigen, dass die MHK auf den Bereich zwischen 1·10⁴ KbE/mL und 5·10⁵ KbE/mL eingegrenzt werden kann. 5·10⁵ KbE/mL entsprechen in den Untersuchung einem Verhältnis von 50 Pilzsporen pro Bakterienzelle.

### Nachweis der antibakteriellen Wirkung von Sporen von Myceliophthora thermophila, wobei der Pilz auf unterschiedlichen Wachstumssubstraten kultiviert wurde, mit dem PREMI-Test

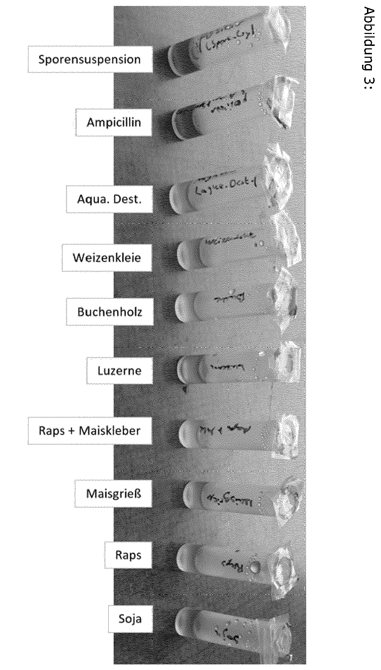

Eine weitere Untersuchung, deren Ergebnisse in Abbildung 3 dargestellt sind, zeigt, dass die Sporen von *Myceliophthora thermophila* dann eine antibakterielle Wirkung haben, wenn sie durch Kultivierung auf Kartoffel-Dextrose-Agar als speziellem Nährmedium hergestellt wurden. Ferner konnten mit einem Wachstumssubstrat, bestehend aus Vermiculit zusammen mit Kartoffelinfus und Glucose, Sporen hergestellt werden, welche die antibakterielle Wirkung ebenfalls zeigten. Eine Lösung aus Kartoffel-Dextrose-Agar und Saline, wie sie zum Ernten der Sporen verwendet wird, zeigte keine antibakterielle Wirkung.

### Nachweis der antibakteriellen Wirkung autoklavierter und nicht-autoklavierter Sporensuspension von Myceliophthora thermophila auf gram-positive Bakterien mit dem 4-Platten-Test

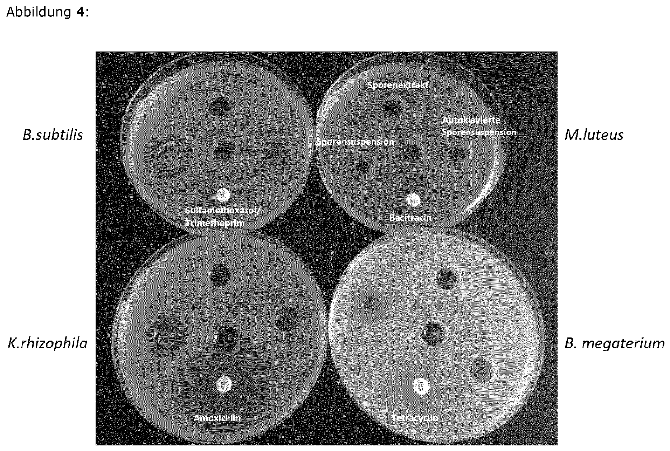

Der 4-Platten-Test ist ein weiteres mikrobiologisches Nachweisverfahren, welches einen Nachweis anti-bakterieller Wirkstoffe ermöglicht. Bei dieser Methode handelt es sich um einen Agardiffusionstest. Dabei werden die zu untersuchenden Testsubstanzen in ausgestanzte Löcher verschiedener Nährmedien gegeben, die mit den Prüfbakterien (Zielbakterien) *Kocuria rhizophila, Micrococcus luteus, Bacillus megaterium* und *Bacillus subtilis* subsp. *spizizenii* beimpft sind. Diese Bakterien sind gram-positiv. Die Anwesenheit antibakteriell wirkender Substanzen wird nach entsprechender Inkubation durch die Bildung von Hemmzonen angezeigt. Dabei handelt es sich jeweils um einen räumlichen Bereich innerhalb des Nähragars, in dem die Zielbakterien nicht wachsen und hierdurch den Agar trüben können. Der Durchmesser des Hemmhofs korreliert mit der Stärke der antibakteriellen Wirkung. Abbildung 4 zeigt die Ergebnisse des 4-Plattentests. Oben rechts ist gezeigt, welche Art von Probe jeweils in die Löcher im Nähragar gegeben wurde. Dabei meint "Sporenextrakt" diejenige Lösung, aus der die Sporen von *Myceliiophthora thermophila* durch Filtration mit einem 0,2-µm-Spritzenfilter entfernt wurden. Die aufgelegten Antibiotikaplättchen dienten als Positivkontrolle. Die Sporensuspension mit Sporen von *Myceliophthora thermophila* zeigte bei allen vier Testbakterien einen wachstumshemmenden Effekt. Nach Autoklavieren zeigte die Sporensuspension von *Myceliophthora thermophila* nur bei *Bacillus subtilis* einen hemmenden Effekt. Die Lösung ohne Pilzsporen zeigte keinen Effekt. Die Anwesenheit der Sporen von *Myceliophthora thermophila* ist demnach für die antibakterielle Wirkung eine Voraussetzung.

### Nachweis der antibakteriellen Wirkung der Sporensuspension von Myceliaphthora thermophila auf Escherichia coli

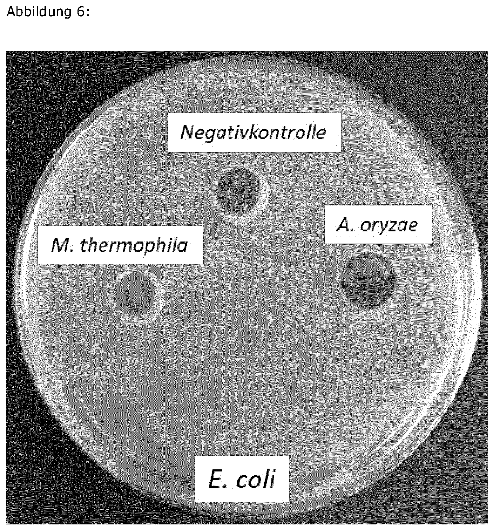

Ein ähnliches Testverfahren wie der 4-Platten-Test wurde verwendet, um die antibakterielle Wirkung der Sporen von *Myceliophthora thermophila* auf gram-negative Bakterien zu untersuchen. Diese Untersuchung unterscheidet sich nur in der verwendeten Bakteriensuspension von *Escherichia coli,* die in den Nähragar eingegossen wurde. Bei *Escherichia coli* handelt es sich um ein gramnegatives Bakterium.
Das Ergebnis ist ein Abbildung 6 dargestellt. Die Sporensuspensionen von *Myceliophthora thermophila* und *Aspergillus oryzae* zeigten keinen wachstumshemmenden Effekt auf *Escherichia coli.*

Auch bei gram-negativen Bakterien der Gattung *Salmonella* konnten mit dem 4-Platten-Test keine antimikrobiellen Effekte beobachtet werden (Ergebnisse nicht gezeigt).

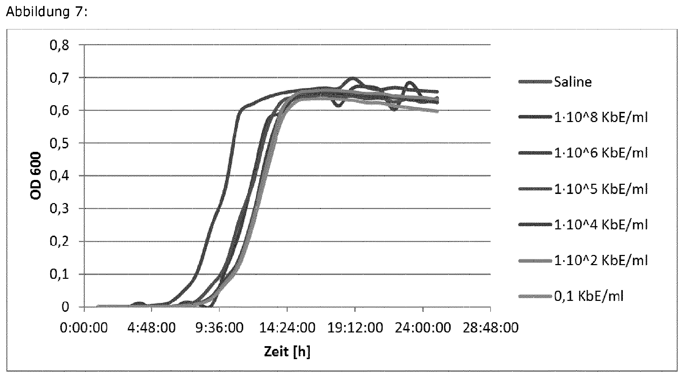

Abbildung 7 zeigt das Ergebnis der Bestimmung der MHK der Sporensuspension von *Myceliophthora thermophila* gegenüber *Escherichia coli,* gemessen mit dem Bakterienwachstumstest mit flüssigem Nährmedium. Auch eine Konzentration der Sporensuspension von 1·10⁸ KbE/mL, angewendet auf eine Bakterienkonzentration von 1·10⁴ KbE/mL, führte nicht zu einer Hemmung des Wachstums von *Escherichia coli.*

### Untersuchung der bakteriziden beziehungsweise bakteriostatischen Wirkung der Sporensuspension von Myceliophthora thermophila

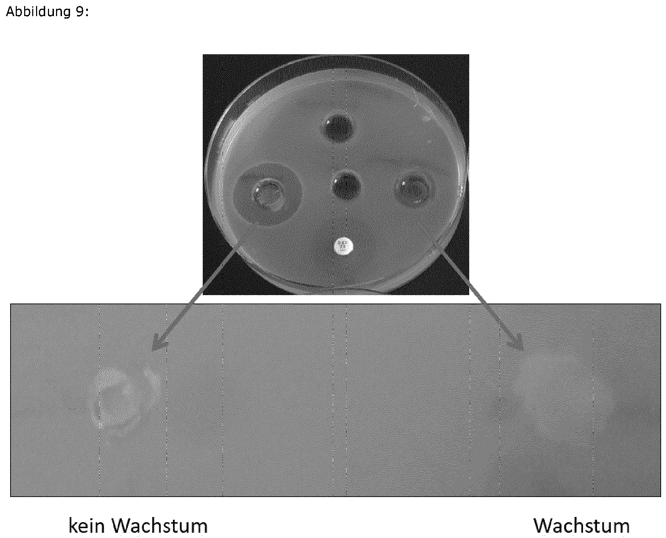

Um zu prüfen, ob die antibakterielle Wirkung der Sporensuspension von *Myceliophthora thermophila* bakterizid oder bakteriostatisch ist, wurde kleines Stück Agar aus dem Bereich des Hemmhofs der Agarplatte mit dem Teststamm *Micrococcus luteus* aus dem 4-Platten-Test (siehe Abbildung 4 zur Orientierung) ausgestanzt und auf eine unbeimpfte CASO-Platte übertragen (siehe Abbildung 9). Als Negativkontrolle wurde ein Stück des bewachsenen Agars außerhalb des Hemmhofs ausgestanzt und auf eine CASO-Platte übertragen. Bei dem aus dem Hemmhof gemäß dem 4-Platten-Test ausgestanzten Agar-Stück fand kein Wachstum in CASO-Medium statt. Bei dem aus dem Bereich außerhalb des Hemmhofs ausgestanzten Agar-Stücks fand Wachstum von *Micrococcus luteus* statt. Dieses Resultat zeigt, dass die Wirkung der Sporensuspension von *Myceliophthora thermophila* bakterizid ist.

### Wirkung inaktivierter Sporensuspension von Myceliophthora thermophila auf Bacillus subtilis

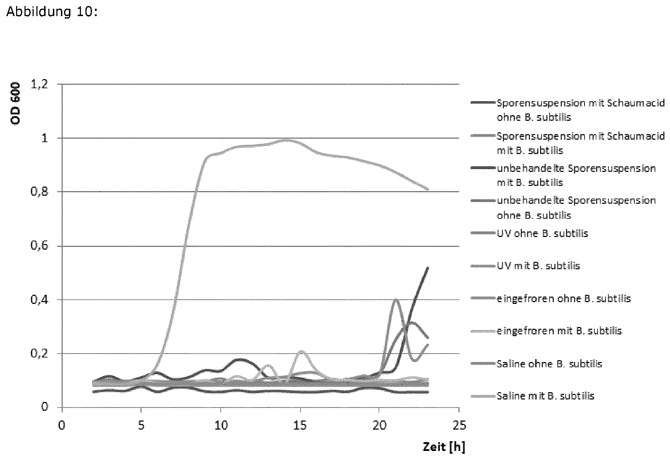

Für diese Untersuchung wurde der Bakterienwachstumstest mit flüssigem Nährmedium angewendet. Dazu wurden die Sporen von *Myceliophthora thermophila* mittels unterschiedlicher Methoden inaktiviert. Die UV-behandelte Sporensuspension wurde 2 h mit UV-Licht bestrahlt. Die mit Säure behandelte Sporensuspension wurde mit 2% Schaumacid (H. Wilhelm Schaumann GmbH, An der Mühlenau 4, 25421 Pinneberg) versetzt, 48 h bei 4°C inkubiert und anschließend neutralisiert. Die eingefrorene Sporensuspension wurde 5 Tage bei -20°C gefroren gehalten. Alle Proben wurden auf Kartoffel-Dextrose-Agar ausplattiert, um zu kontrollieren, ob die Sporen tatsächlich abgetötet wurden und somit nicht mehr auskeimten. Von den verschiedenen Behandlungen erhielt nur die Behandlung der Sporen mit dem Säurepräparat (Schaumacid) die antibaktierelle Wirkung gegenüber *Bacillus subtilis* (siehe Abbildung 10). Dabei spielte es keine Rolle, ob die Probe während der Behandlung bei 4°C oder bei 30°C gelagert worden war (Ergebnisse nicht dargestellt).

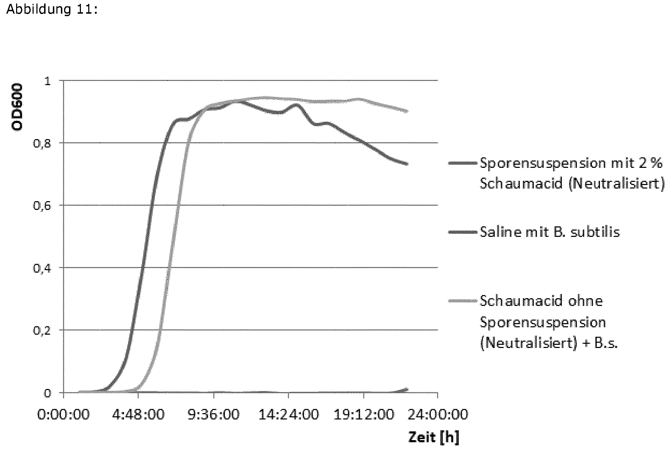

Zum Zweck der Kontrolle wurde untersucht, ob die antibakterielle Wirkung nicht durch das Säurepräparat selbst verursacht worden war. Dazu wurde Salinelösung mit Schaumacid (2%ig) versetzt, anschließend neutralisiert und zu einer Bakteriensuspension von *Bacillus subtilis* gegeben. Wie Abbildung 11 zeigt, hatte Schaumacid alleine keine antibakterielle Wirkung gegenüber *Bacillus substilis.*

### Wirkung von gefriergetrockneten Sporen von Myceliophthora thermophila auf Bacillus subtilis

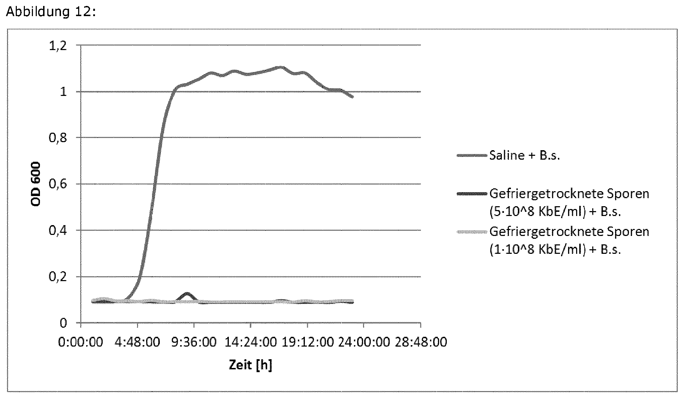

Zur Haltbarmachung der Sporen von *Myceliophthora thermophila* erwies sich die Gefriertrocknung als geeignet. Eine Untersuchung mit gefriergetrockneten Sporen mittels des Bakterienwachstumstests mit flüssigem Nährmedium zeigte, dass - auch bei vorheriger Behandlung mit 2% Schaumacid - die antibakterielle Wirkung der Sporen gegenüber *Bacillus subtilis* weiterhin bestehen blieb (siehe Abbildung 12). Die MHK wird durch das Gefriertrocknen nicht geändert.

### Wirkung der Sporen aus der dritten Generation von Myceliophthora thermophila auf Bacillus subtilis und Kocuria rhizophila

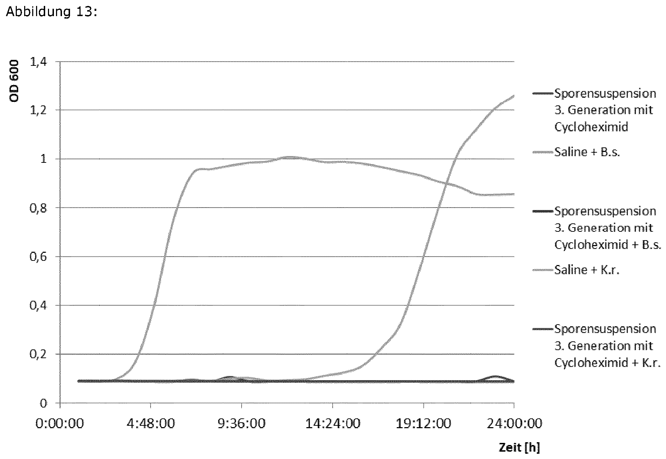

Für die bisher beschriebenen Untersuchungen wurde eine Sporensuspension der zweiten Generation von *Myceliophthora thermophila* verwendet. Um zu untersuchen, ob die antibakterielle Wirkung auch länger bestehen bleibt, wurden Sporen der dritten Generation mittels des Bakterienwachstumsassays mit flüssigem Nährmedium mit *Bacillus substilils* und *Kocuria rhizophila* untersucht.
Die Ergebnisse in Abbildung 13 zeigen, dass auch die Pilzsporen der dritten Generation die antibakterielle Wirkung gegenüber *Bacillus substilis* und *Kocuria rhizophila* zeigten.

### Lagerstabilität der antibakteriellen Wirkung der Sporen von Myceliophthora thermophila

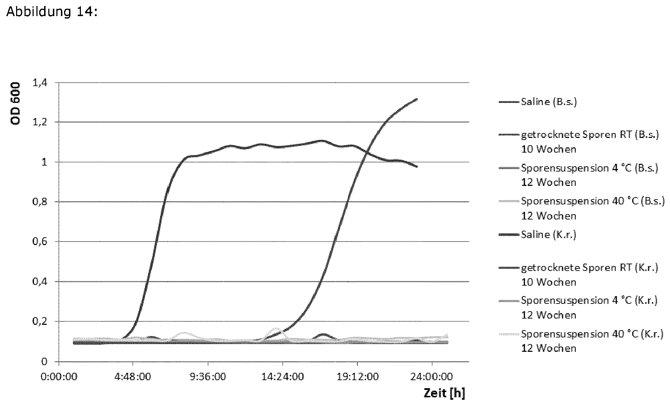

Die in Abbildung 14 dargestellten Ergebnisse einer weiteren Untersuchung mittels des Bakterienwachstumstests mit flüssigem Nährmedium zeigen, dass die antibakterielle Wirkung gefriergetrockneter Sporen von *Myceliophthora thermophila* gegenüber *Bacillus subtilis* und *Kocuria rhizophila* bei Lagerung bei Raumtemperatur über einen Zeitraum von 12 Wochen stabil blieb. Bei 40°C konnten bewachsene PDA-Kolben über 20 Wochen stabil gelagert werden. Auch die Lagerung der Sporensuspension bei 4°C über 20 Wochen ergab keine Abnahme der antibakteriellen Wirkung.

### Externe Studie zur antibakteriellen Wirkung der Sporen von Myceliophthora thermophila

Die antibakterielle Wirkung der Sporen von *Myceliophthora thermophila* gegenüber grampositiven Bakterien wurde durch eine standardisierte Studie in einem externen Labor bestätigt. Diese Studie ist in der Anlage beigefügt.

### Anlage:

### Externe Studie zur antibakteriellen Wirkung der Sporen von Myceliophthora thermophila

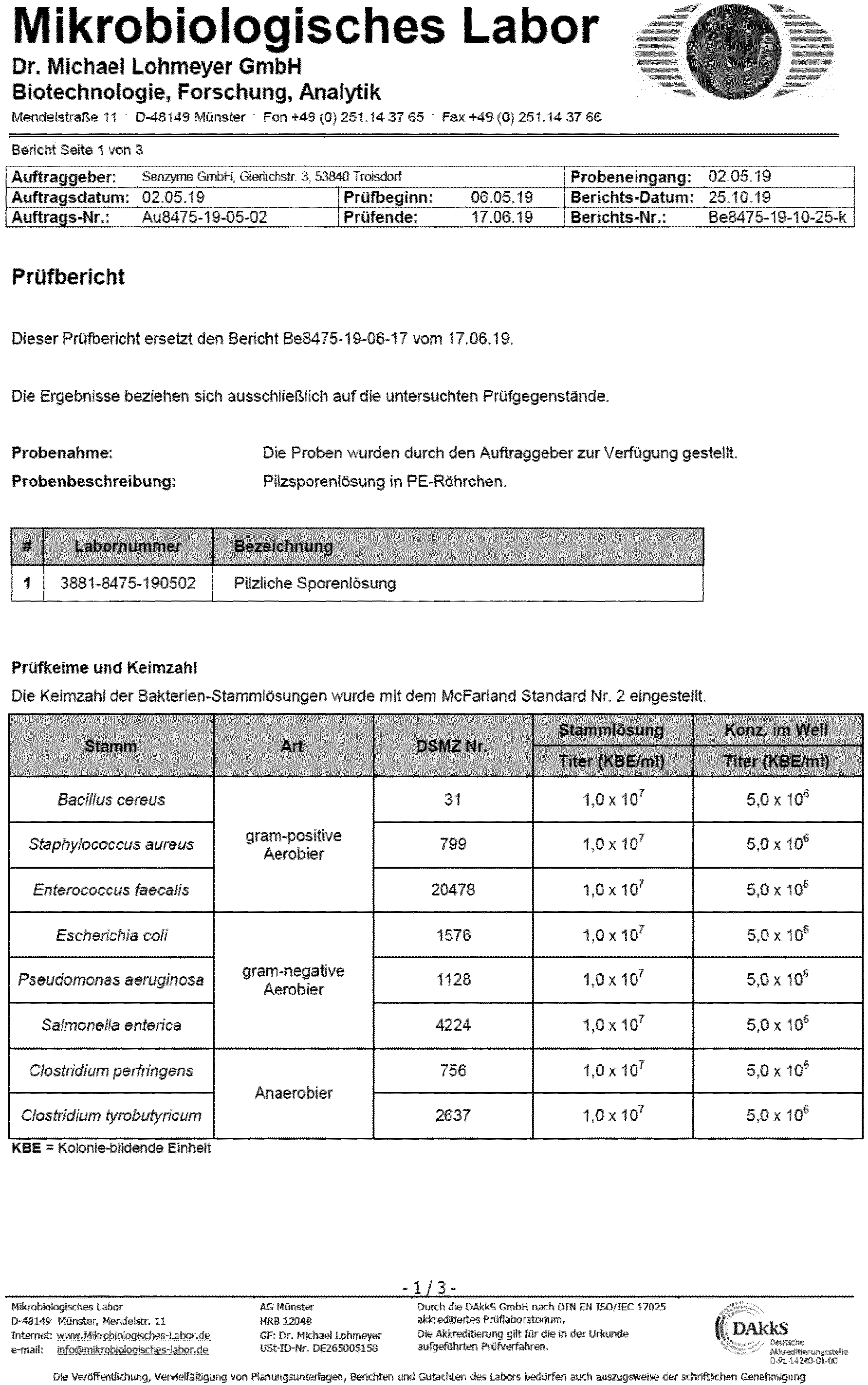

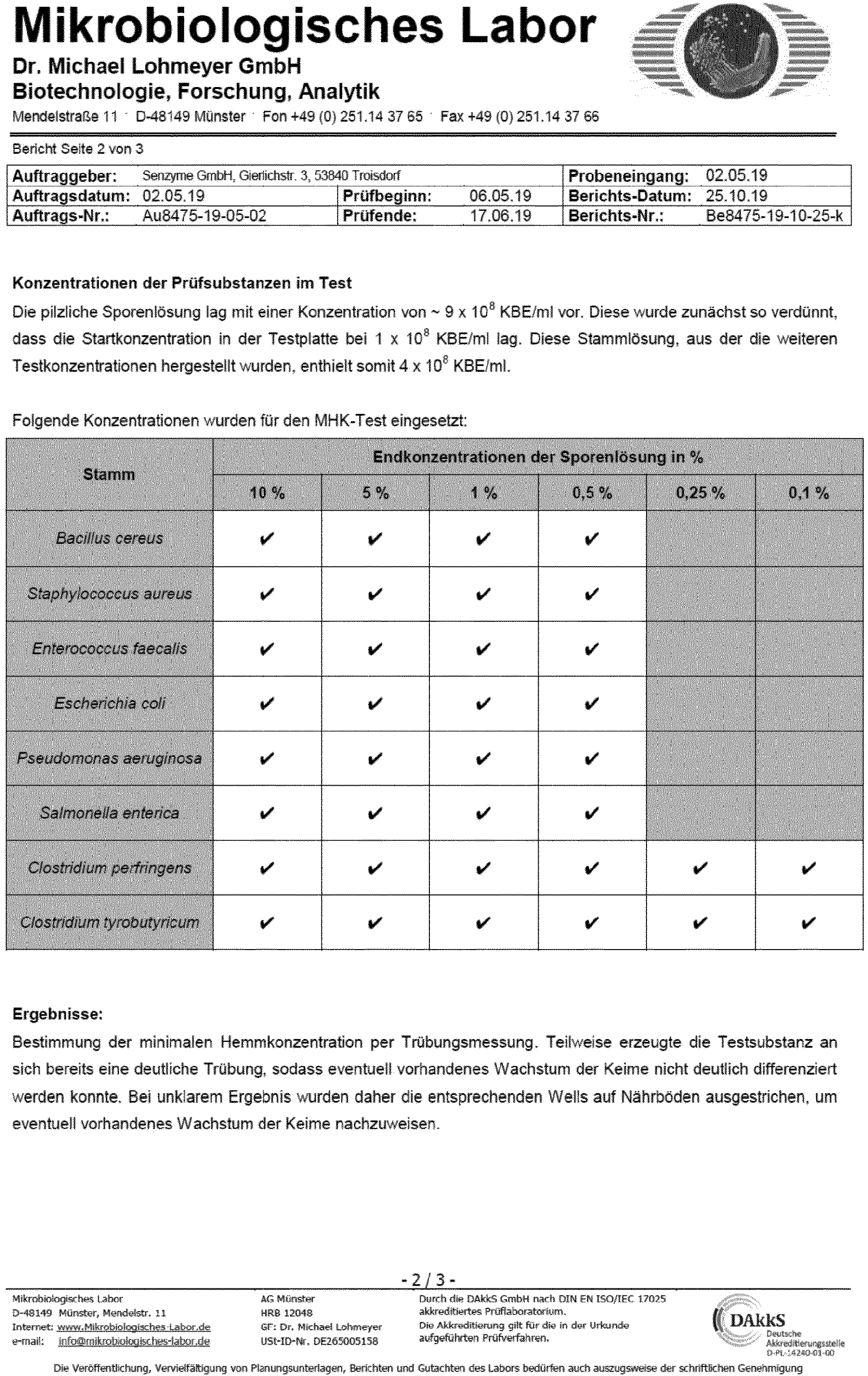

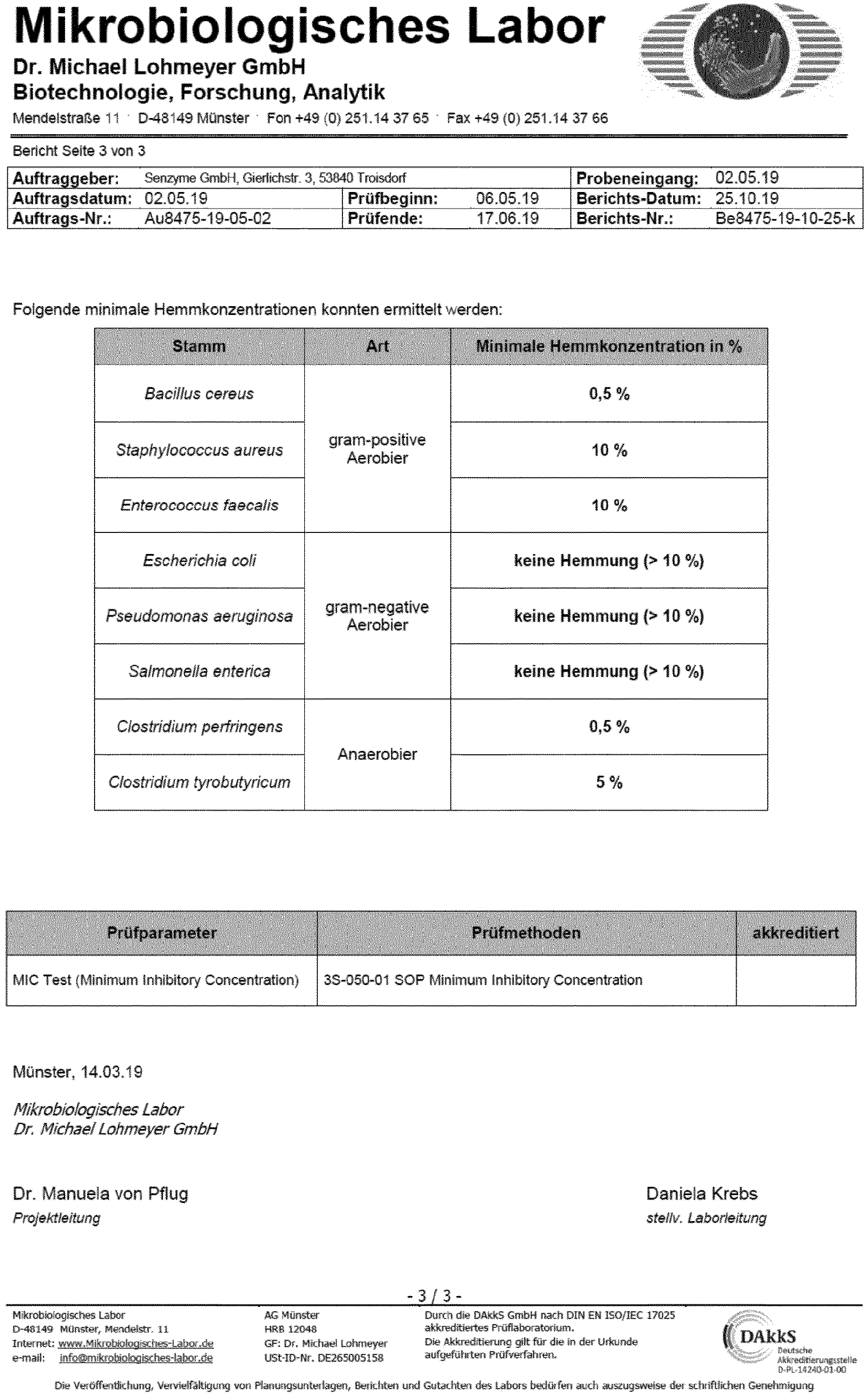

## Patentansprüche

1. Verwendung eines bakteriziden Mittels für gram-positive Bakterien enthaltend Sporen des Pilzes Myceliophthora thermophila zur nicht-therapeutischen Leistungssteigerung von Wirbeltieren und/oder Menschen durch die Stabilisierung der Darmflora.

2. Verwendung eines bakteriziden Mittels für gram-positive Bakterien enthaltend Sporen des Pilzes Myceliophthora thermophila zur nicht-therapeutischen Förderung des Wohlbefindens von Wirbeltieren und/oder Menschen durch die Stabilisierung der Bakterienflora von Hautoberflächen, Körperöffnungen, Mund (bzw. Maul), Rachen oder anderen äußeren oder inneren Körperoberflächen.

3. Verwendung eines bakteriziden Mittels für gram-positive Bakterien enthaltend Sporen des Pilzes Myceliophthora thermophila als Desinfektionsmittel und//oder als Konservierungsmittel für die Behandlung fester oder flüssiger Materialien zum Verbessern der Hygiene im Umfeld von Wirbeltieren und/oder Menschen.

4. Verwendung eines bakteriziden Mittels für gram-positive Bakterien enthaltend Sporen des Pilzes Myceliophthora thermophila als Mittel zur nicht-therapeutischen Körperhygiene bei der Pflege von äußeren oder inneren Körperoberflächen bei Wirbeltieren und/oder Menschen.

5. Verwendung nach einem der Ansprüche 1 bis 4, wobei das bakterizide Mittel inaktivierte Sporen des Pilzes M. thermophila enthält.

6. Verwendung nach Anspruch 5, wobei das bakterizide Mittel eine Säure und/oder mit UV-Licht inaktivierte Sporen des Pilzes M. thermophila enthält.

7. Verwendung nach einem der Ansprüche 1 bis 6, wobei das bakterizide Mittel gefriergetrocknete, vakuumgetrocknete oder sprühgetrocknete Sporen des Pilzes M. thermophila enthält.

8. Verwendung nach einem der Ansprüche 1 bis 7, wobei das bakterizide Mittel einen Trägerstoff und Sporen des Pilzes M. thermophila enthält.

9. Verwendung nach einem der Ansprüche 1 bis 8 mit dem bakteriziden Mittel in Form eines Feststoffes.

10. Verwendung nach einem der Ansprüche 1 bis 8 mit dem bakteriziden Mittel in einer Formulierung als Paste oder Creme.

11. Verwendung nach einem der Ansprüche 1 bis 8 mit dem bakteriziden Mittel in einer flüssigen Formulierung.

12. Verwendung nach Anspruch 11 mit dem bakteriziden Mittel in einer flüssigen Formulierung auf der Basis von Wasser oder von Alkohol.

13. Verwendung nach einem der Ansprüche 1 bis 12 mit dem bakteriziden Mittel als Futtermittelzusatzstoff oder als Lebensmittelzusatzstoff.

14. Verwendung nach Anspruch 13 mit dem bakteriziden Mittel als Futtermittelzusatzstoff in einem Futtermittel enthalten oder als Lebensmittelzusatzstoff in einem Lebensmittel enthalten

15. Verwendung nach einem der Ansprüche 1 bis 14, bei dem das Mittel dem Trinkwasser zugesetzt und das Trinkwasser Wirbeltieren und/oder Menschen verabreicht wird.

16. Verwendung nach einem der Ansprüche 1 bis 15, bei dem das Mittel einem Futtermittel oder Lebensmittel zugesetzt und das Futtermittel Wirbeltieren oder das Lebensmittel Menschen verabreicht wird.

17. Verwendung nach Anspruch 3, bei dem das Mittel dem Trinkwasser zugesetzt und Oberflächen im Stall, Käfig, Gehege oder in anderen Bereichen, die der Haltung von Nutztieren oder Haustieren dienen, mit dem Trinkwasser behandelt werden.

18. Verwendung nach Anspruch 17, bei dem die Oberflächen von Tränken und/oder von Futtertrögen und/oder die Einstreu von Stallungen mit dem Mittel behandelt werden.

19. Verwendung nach Anspruch 3, bei dem das Mittel zum Konservieren einem Futtermittel oder Lebensmittel zugesetzt wird.

20. Verwendung nach einem der Ansprüche 1 bis 19, bei dem das Mittel zum Abtöten oder Inhibieren mindestens eines der folgenden grampositiven Bakterien verwendet wird: Bacillus subtilis, Bacillus stearothermophilus, Bacillus cereus, Staphylococcus aureus, Enterococcus faecalis, Clostridium perfringens, Clostridium tyrobutyricum.

21. Bakterizides Mittel für gram-positive Bakterien enthaltend Sporen des Pilzes Myceliophthora thermophila zur Anwendung in einem Verfahren zur Behandlung oder Vorbeugung bakterieller Erkrankungen durch gram-positive Bakterien, insbesondere von Darmerkrankungen und/oder von Entzündungen der Schleimhäute beim Menschen und/oder bei Tieren.

22. Mittel nach Anspruch 21, das ein bakterizides Mittel aus einem der Ansprüche 5 bis 13 ist.

## Claims

1. A use of a bactericidal agent for gram-positive bacteria containing spores of the fungus Myceliophthora thermophila for non-therapeutically increasing the performance of vertebrates and/or humans by stabilizing the intestinal flora.

2. A use of a bactericidal agent for gram-positive bacteria containing spores of the fungus Myceliophthora thermophila for non-therapeutically promoting the well-being of vertebrates and/or humans by stabilizing the bacterial flora of skin surfaces, body orifices, mouth (or else muzzle), throat, or other external or internal body surfaces.

3. A use of a bactericidal agent for gram-positive bacteria containing spores of the fungus Myceliophthora thermophila as a disinfectant and/or as a preservative for treating solid or liquid materials in order to improve hygiene in the environment of vertebrates and/or humans.

4. A use of a bactericidal agent for gram-positive bacteria containing spores of the fungus Myceliophthora thermophila as a non-therapeutic body hygiene agent for caring for external or internal body surfaces in vertebrates and/or humans.

5. The use according to any one of claims 1 to 4, wherein the bactericidal agent contains inactivated spores of the fungus M. thermophila.

6. The use according to claim 5, wherein the bactericidal agent contains an acid and/or spores of the fungus M. thermophila that have been inactivated by means of UV light.

7. The use according to any one of claims 1 to 6, wherein the bactericidal agent contains freeze-dried, vacuum-dried, or spray-dried spores of the fungus M. thermophila.

8. The use according to any one of claims 1 to 7, wherein the bactericidal agent contains a carrier and spores of the fungus M. thermophila.

9. The use according to any one of claims 1 to 8, with the bactericidal agent in the form of a solid.

10. The use according to any one of claims 1 to 8, with the bactericidal agent in a formulation as a paste or cream.

11. The use according to any one of claims 1 to 8, with the bactericidal agent in a liquid formulation.

12. The use according to claim 11, with the bactericidal agent in a liquid formulation based on water or alcohol.

13. The use according to any one of claims 1 to 12, with the bactericidal agent as a feed additive or food additive.

14. The use according to claim 13, with the bactericidal agent as a feed additive contained in a feed or as a food additive contained in a food.

15. The use according to any one of claims 1 to 14, wherein the agent is added to drinking water and the drinking water is administered to vertebrates and/or humans.

16. The use according to any one of claims 1 to 15, wherein the agent is added to a feed or food and the feed is administered to vertebrates or the food is administered to humans.

17. The use according to claim 3, wherein the agent is added to drinking water and surfaces in barns, cages, enclosures, or in other regions used for keeping livestock or pets are treated with the drinking water.

18. The use according to claim 17, wherein the surfaces of drinking troughs and/or feed troughs and/or stable bedding are treated with the agent.

19. The use according to claim 3, wherein the agent is used to preserve a feed or food.

20. The use according to any one of claims 1 to 19, wherein the agent is used to kill off or inhibit at least one of the following gram-positive bacteria: Bacillus subtilis, Bacillus stearothermophilus, Bacillus cereus, Staphylococcus aureus, Enterococcus faecalis, Clostridium perfringens, Clostridium tyrobutyricum.

21. A bactericidal agent for gram-positive bacteria containing spores of the fungus Myceliophthora thermophila for use in a method for treating or preventing bacterial diseases caused by gram-positive bacteria, in particular intestinal diseases and/or inflammation of the mucous membranes in humans and/or animals.

22. The agent according to claim 21, which is a bactericidal agent from any one of claims 5 to 13.

## Revendications

1. Utilisation d'un agent bactéricide pour des bactéries à Gram positif contenant des spores du champignon Myceliophthora thermophila pour l'augmentation non thérapeutique des performances de vertébrés et/ou d'humains par stabilisation de la flore intestinale.

2. Utilisation d'un agent bactéricide pour des bactéries à Gram positif contenant des spores du champignon Myceliophthora thermophila pour l'amélioration non thérapeutique du bien-être de vertébrés et/ou d'humains par stabilisation de la flore bactérienne de surfaces cutanées, de cavités corporelles, de la bouche (ou de la gueule), de la gorge ou d'autres surfaces corporelles extérieures ou intérieures.

3. Utilisation d'un agent bactéricide pour des bactéries à Gram positif contenant des spores du champignon Myceliophthora thermophila comme agent désinfectant et/ou comme agent conservateur pour le traitement de matières solides ou liquides pour l'amélioration de l'hygiène dans l'environnement de vertébrés et/ou d'humains.

4. Utilisation d'un agent bactéricide pour des bactéries à Gram positif contenant des spores du champignon Myceliophthora thermophila comme agent pour l'hygiène corporelle non thérapeutique pendant le soin de surfaces corporelles extérieures ou intérieures chez des vertébrés et/ou des humains.

5. Utilisation selon l'une des revendications 1 à 4, dans laquelle l'agent bactéricide contient des spores inactivées du champignon M. thermophila.

6. Utilisation selon la revendication 5, dans laquelle l'agent bactéricide contient un acide et/ou des spores inactivées par lumière ultraviolette du champignon M. thermophila.

7. Utilisation selon l'une des revendications 1 à 6, dans laquelle l'agent bactéricide contient des spores lyophilisées, séchées sous vide ou séchées par pulvérisation du champignon M. thermophila.

8. Utilisation selon l'une des revendications 1 à 7, dans laquelle l'agent bactéricide contient un support et des spores du champignon M. thermophila.

9. Utilisation selon l'une des revendications 1 à 8, avec l'agent bactéricide sous la forme d'une matière solide.

10. Utilisation selon l'une des revendications 1 à 8, avec l'agent bactéricide dans une formulation en pâte ou crème.

11. Utilisation selon l'une des revendications 1 à 8, avec l'agent bactéricide dans une formulation liquide.

12. Utilisation selon la revendication 11, avec l'agent bactéricide dans une formulation liquide à base d'eau ou d'alcool.

13. Utilisation selon l'une des revendications 1 à 12, avec l'agent bactéricide en tant qu'additif d'aliments pour animaux ou en tant qu'additif de denrée alimentaire

14. Utilisation selon la revendication 13, avec l'agent bactéricide en tant qu'additif d'aliment pour animaux contenu dans un aliment pour animaux ou en tant qu'additif de denrée alimentaire contenu dans une denrée alimentaire.

15. Utilisation selon l'une des revendications 1 à 14, dans laquelle l'agent est ajouté à l'eau potable et l'eau potable est donnée à des vertébrés et/ou à des humains.

16. Utilisation selon l'une des revendications 1 à 15, dans laquelle l'agent est ajouté à un aliment pour animaux ou à une denrée alimentaire et l'aliment pour animaux est donné à des vertébrés ou la denrée alimentaire est donnée à des humains.

17. Utilisation selon la revendication 3, dans laquelle l'agent est ajouté à l'eau potable et des surfaces dans une étable, une cage, un enclos ou d'autres zones servant à l'élevage de bétail ou d'animaux domestiques sont traitées avec l'eau potable.

18. Utilisation selon la revendication 17, dans laquelle les surfaces d'abreuvoirs et/ou de mangeoires et/ou la litière de stabulations sont traitées avec l'agent.

19. Utilisation selon la revendication 3, dans laquelle l'agent est ajouté à un aliment pour animaux ou à une denrée alimentaire pour la conservation.

20. Utilisation selon l'une des revendications 1 à 19, dans laquelle l'agent est utilisé pour tuer ou inhiber l'une au moins des bactéries à Gram positif suivantes : Bacillus subtilis, Bacillus stearothermophilus, Bacillus cereus, Staphylococcus aureus, Enterococcus faecalis, Clostridium perfringens, Clostridium tyrobutyricum.

21. Agent bactéricide pour des bactéries à Gram positif contenant des spores du champignon Myceliophthora thermophila, destiné à être utilisé dans un procédé de traitement ou de prévention de maladies bactériennes dues à des bactéries à Gram positif, en particulier de maladies intestinales et/ou d'inflammations des muqueuses chez l'humain et/ou chez des animaux.

22. Agent selon la revendication 21, lequel est un agent bactéricide selon l'une des revendications 5 à 13.
